# EUROPEAN PATENT APPLICATION

(11) **EP 4 148 067 A1**
(43) Date of publication of application: **15.03.2023**
(21) Application number: 21195415.1
(22) Date of filing: 08.09.2021
(51) Int. Cl.: C07K 16/00, C07K 16/40, C07K 14/525, C12N 15/62

(54) **METHOD FOR THE EXPRESSION OF AN ANTIBODY-MULTIMER-FUSION**

(71) Applicant: F. Hoffmann-La Roche AG, 4070 Basel (CH); GENENTECH, INC., South San Francisco, CA 94080 (US)
(72) Inventor: CHUNG, Shan-Hua, 82377 Penzberg (DE); DUERR, Harald, 82377 Penzberg (DE); TOURNAVITI, Styliani, 82377 Penzberg (DE); FERRARA KOLLER, Claudia, 8952 Schlieren (CH); MISAGHI, Shahram, 94080 South San Francisco (US); SHEN, Amy, 94080 South San Francisco (US); YIP, Shuet Ming, 94080 South San Francisco (US)
(74) Representative: Burger, Alexander

(57) **Abstract**

Herein is reported a method for producing an antibody-multimer-fusion polypeptide comprising
(a) an antibody heavy chain and an antibody light chain, and
(b) a first fusion polypeptide comprising in N- to C-terminal direction a first part of a non-antibody multimeric polypeptide, an antibody heavy chain CH1 domain or an antibody light chain constant domain, an antibody hinge region, an antibody heavy chain CH2 domain and an antibody heavy chain CH3 domain, and a second fusion polypeptide comprising in N- to C-terminal direction the second part of the non-antibody multimeric polypeptide and an antibody light chain constant domain if the first polypeptide comprises an antibody heavy chain CH1 domain or an antibody heavy chain CH1 domain if the first polypeptide comprises an antibody light chain constant domain,
wherein (i) the antibody heavy chain of (a) and the first fusion polypeptide of (b), (ii) the antibody heavy chain of (a) and the antibody light chain of (a), and (iii) the first fusion polypeptide of (b) and the second fusion polypeptide of (b) are each independently of each other covalently linked to each other by at least one disulfide bond,
characterized in that the antibody-multimer-fusion is expressed by a recombinant mammalian cell obtained by transfecting a mammalian cell with the expression cassettes for the antibody heavy chain, the antibody light chain, the first fusion polypeptide and the second fusion polypeptide at a stoichiometric ratio of 1:1:2:1.

## Description

The current invention is in the field of cell line generation and polypeptide production. More precisely, herein is reported the generation of a recombinant mammalian production cell by transfecting a non-producing mammalian cell with a defined ratio of the expression cassettes for the individual polypeptides of the antibody-multimer-fusion. Said cell can be used in a method for the production of an antibody-multimer-fusion.

### Background of the Invention

Secreted and glycosylated polypeptides, such as e.g. antibodies, are usually produced by recombinant expression in eukaryotic cells, either as stable or as transient expression.

The higher the complexity of the polypeptide to be produced, i.e. the higher the number of different polypeptides or polypeptide chains required to form the polypeptide of interest inside the cell, the more important becomes the control of the expression ratio of the different polypeptides or polypeptide chains relative to each other. The control of the expression ratio is required to enable efficient expression, correct assembly and successful secretion in high expression yield of the polypeptide of interest.

One strategy for generating a recombinant cell expressing an exogenous polypeptide of interest involves the random integration of a nucleotide sequence encoding the polypeptide of interest followed by selection and isolation steps.

Targeted integration by recombinase mediated cassette exchange (RMCE) is a method to direct foreign DNA specifically and efficiently to a pre-defined site in a eukaryotic host genome (Turan et al., J. Mol. Biol. 407 (2011) 193-221).

Crawford et al. reported a fast identification of reliable hosts for targeted cell line development from a limited-genome screening using combined ϕC31 integrase and CRE-Lox technologies (Biotechnol. Prog. 29 (2013) 1307-1315).

Rajendra et alRajendra et al. reported that a single quad vector is a simple, yet effective, alternative approach for generation of stable CHO cell lines and may accelerate cell line generation for clinical hetero-mAb therapeutics (Biotechnol. Prog. 33 (2017) 469-477).

Bahr et al. reported the development of a platform expression system using targeted integration in Chinese hamster ovary cells (proceedings of Cell Culture Engineering XVI, 2018).

Carver et al. reported maximizing antibody production in a targeted integration host by optimization of subunit gene dosage and position (Biotechnol. Prog. (2020) e2967).

### Summary of the Invention

The current invention is defined by the following independent aspects and dependent embodiments:
1. A first aspect of the invention is a method for producing an antibody-multimer-fusion polypeptide
   comprising
      (a) an antibody heavy chain and an antibody light chain, and
      (b) a first fusion polypeptide comprising in N- to C-terminal direction a first part of a non-antibody multimeric polypeptide, an antibody heavy chain CH1 domain or an antibody light chain constant domain, an antibody hinge region, an antibody heavy chain CH2 domain and an antibody heavy chain CH3 domain, and a second fusion polypeptide comprising in N- to C-terminal direction the second part of the non-antibody multimeric polypeptide and an antibody light chain constant domain if the first polypeptide comprises an antibody heavy chain CH1 domain or an antibody heavy chain CH1 domain if the first polypeptide comprises an antibody light chain constant domain,
      wherein
      (i) the antibody heavy chain of (a) and the first fusion polypeptide of (b), (ii) the antibody heavy chain of (a) and the antibody light chain of (a), and (iii) the first fusion polypeptide of (b) and the second fusion polypeptide of (b) are each independently of each other covalently linked to each other by at least one disulfide bond,
      wherein
      the variable domains of the antibody heavy chain and the antibody light chain form a binding site specifically binding to an antigen,
   characterized in that the antibody-multimer-fusion polypeptide is expressed by a recombinant mammalian cell obtained by transfecting a (parent) mammalian cell with the expression cassettes for the antibody heavy chain, the antibody light chain, the first fusion polypeptide and the second fusion polypeptide at a stoichiometric ratio of 1:1:2:1.
2. The method according to aspect 1, wherein the antibody-multimer-fusion polypeptide is transiently or stably expressed.
3. The method according to any one of aspect 1 or embodiment 2, wherein the mammalian cell is a CHO cell, preferably a CHO-K1, or a HEK cell.
4. The method according to any one of aspect 1 and embodiments 2 to 3, wherein the transfecting is of four vectors, whereby each vector comprises exactly one of the expression cassettes.
5. The method according to any one of aspect 1 and embodiments 2 to 3, wherein the transfecting is of three vectors, whereby two vectors comprise exactly two of the expression cassettes and one vector comprises exactly one of the expression cassettes.
6. The method according to embodiment 4, wherein the transfecting is of three vectors, whereby the first vector comprises the expression cassettes for the an antibody heavy chain and an antibody light chain, the second expression vector comprises the expression cassettes for the first fusion polypeptide and the second fusion polypeptide, and the third vector comprises one expression cassette for the first fusion polypeptide.
7. The method according to any one of aspect 1 and embodiments 2 to 6, wherein the first fusion polypeptide comprises as first part of the non-antibody multimeric polypeptide two ectodomains of a TNF ligand family member or a fragment thereof that are connected to each other by a peptide linker, and the second fusion polypeptide comprises as second part of a non-antibody multimeric polypeptide only one ectodomain of said TNF ligand family member or a fragment thereof, or vice versa.
8. The method according to embodiment 7, wherein
   (a) the first fusion polypeptide comprises as first part of the non-antibody multimeric polypeptide a first ectodomain of a TNF ligand family member or a fragment thereof, a spacer domain and a second ectodomain of said TNF ligand family member or a fragment thereof, wherein
      - the spacer domain is a polypeptide and comprises at least 25 amino acid residues,
      - the first ectodomain of a TNF ligand family member or a fragment thereof is fused either directly or via a first peptide linker to the N-terminus of the spacer domain and
      - the second ectodomain of said TNF ligand family member or a fragment thereof is fused either directly or via a second peptide linker to the C-terminus of the spacer domain,
   (b) the second fusion polypeptide comprises as second part of the non-antibody multimeric polypeptide a third ectodomain of said TNF ligand family member or a fragment thereof that is fused either directly or via a third peptide linker to
      - either the C-terminus of the second ectodomain of said TNF ligand family member in the first fusion polypeptide or to the C-terminus of the spacer domain in the second fusion polypeptide, or
      - in case the second part of the antigen binding domain is fused to the C-terminus of the spacer domain of the second fusion protein, to the C-terminus of the second ectodomain of said TNF ligand family member in the first fusion polypeptide.
9. The method according to any one of aspect 1 and embodiments 2 to 8, wherein
   the first fusion polypeptide comprises in N- to C-terminal direction a first part of a non-antibody multimeric polypeptide, an antibody light chain constant domain, an antibody hinge region, an antibody heavy chain CH2 domain and an antibody heavy chain CH3 domain, and
   the second fusion polypeptide comprising in N- to C-terminal direction the second part of the non-antibody multimeric polypeptide and an antibody heavy chain CH1 domain.
10. The method according to any one of aspect 1 and embodiments 2 to 9, wherein
   the first fusion polypeptide comprises the knob mutation, and
   the antibody heavy chain comprises the hole mutations.
11. The method according to any one of aspect 1 and embodiments 2 to 10, wherein the antibody heavy chain of (a) and the first fusion polypeptide of (b) form an Fc-region.
12. The method according to any one of aspect 1 and embodiments 2 to 11, wherein the antibody heavy chain of (a) and the first fusion polypeptide of (b) form an IgG1 Fc-region or an IgG4 Fc-region.
13. The method according to any one of aspect 1 and embodiments 2 to 12, wherein the Fc-region is an IgG1 Fc-region further comprising the amino acid substitutions at positions 234 and 235 and/or 329 (Kabat EU numbering).
14. The method according to any one of aspect 1 and embodiments 2 to 13, wherein the Fc-region is an IgG1 Fc-region further comprising the amino acid substitutions L234A, L235A and/or P329G (Kabat EU numbering).
15. The method according to any one of embodiments 7 to 14, wherein in the first fusion polypeptide the two ectodomains of a TNF ligand family member or fragments thereof connected to each other by a first peptide linker and are fused at its C-terminus by a second peptide linker to a CH1 domain, and in the second fusion polypeptide the one ectodomain of said TNF ligand family member or a fragment thereof is fused at its C-terminus by a third peptide linker to the antibody light chain constant domain.
16. The method according to any one of embodiments 7 to 14, wherein in the first fusion polypeptide the two ectodomains of a TNF ligand family member or fragments thereof connected to each other by a first peptide linker and are fused at its C-terminus by a second peptide linker to light chain constant domain, and in the second fusion polypeptide the one ectodomain of said TNF ligand family member or a fragment thereof is fused at its C-terminus by a third peptide linker to the a heavy chain CH1 domain.
17. The method according to any one of aspect 1 and embodiments 2 to 16, wherein in the CL domain adjacent to the part of a non-antibody multimeric polypeptide the amino acid at position 123 (Kabat EU numbering) has been replaced by arginine (R) and the amino acid at position 124 (Kabat EU numbering) has been substituted by lysine (K), and wherein in the CH1 domain adjacent to the part of a non-antibody multimeric polypeptide the amino acids at position 147 (Kabat EU numbering) and at position 213 (Kabat EU numbering) have been substituted by glutamic acid (E).
18. The method according to any one of aspect 1 and embodiments 2 to 17, wherein the variable domains of the antibody heavy chain and the antibody light chain form a binding site specifically binding to a cell surface antigen.
19. The method according to any one of aspect 1 and embodiments 2 to 18, wherein the variable domains of the antibody heavy chain and the antibody light chain form a binding site specifically binding to a cell surface antigen selected from the group consisting of Fibroblast Activation Protein (FAP), Melanoma-associated Chondroitin Sulfate Proteoglycan (MCSP), Epidermal Growth Factor Receptor (EGFR), Carcinoembryonic Antigen (CEA), CD19, CD20 and CD33.
20. The method according to any one of embodiments 7 to 19, wherein the TNF ligand family member co-stimulates human T-cell activation.
21. The method according to any one of embodiments 7 to 20, wherein the TNF ligand family member is selected from 4-1-BBL and OX40L.
22. The method according to any one of embodiments 7 to 21, wherein the TNF ligand family member is 4-1-BBL.
23. The method according to any one of embodiments 7 to 22, wherein the ectodomain of a TNF ligand family member comprises the amino acid sequence selected from the group consisting of SEQ ID NO: 01, SEQ ID NO: 02, SEQ ID NO: 03, SEQ ID NO: 04, SEQ ID NO: 56, SEQ ID NO: 100, SEQ ID NO: 101 and SEQ ID NO: 102.
24. The method according to any one of embodiments 7 to 23, wherein the ectodomain of a TNF ligand family member comprises the amino acid sequence of SEQ ID NO: 01 or SEQ ID NO: 56.
25. The method according to any one of aspect 1 and embodiments 2 to 24, wherein
   (a) the antibody heavy chain and the antibody light chain form a binding site capable of specific binding to a target cell antigen, and
   (b) the first fusion polypeptide comprises an amino acid sequence selected from the group consisting of SEQ ID NO: 05, SEQ ID NO: 57, SEQ ID NO: 58 and SEQ ID NO: 59, and the second fusion polypeptide comprises an amino acid sequence selected from the group consisting of SEQ ID NO: 01, SEQ ID NO: 56, SEQ ID NO: 03 and SEQ ID NO: 04.
26. The method according to any one of embodiments 7 to 21, wherein the TNF ligand family member is OX40L.
27. The method according to any one of embodiments 7 to 21 and 26, wherein the ectodomain of a TNF ligand family member comprises the amino acid sequence of SEQ ID NO: 43 or SEQ ID NO: 44, particularly the amino acid sequence of SEQ ID NO: 43.
28. The method according to any one of embodiments 7 to 21 and 26 to 27, wherein the antibody-multimer-fusion comprises
   (a) at least one moiety capable of specific binding to a target cell antigen, and
   (b) the first and the second fusion polypeptide are linked to each other by a disulfide bond, wherein the antigen binding molecule is characterized in that the first fusion polypeptide comprises the amino acid sequence of SEQ ID NO: 99 or SEQ ID: 100 and in that the second fusion polypeptide comprises the amino acid sequence of SEQ ID NO: 43 or SEQ ID NO: 44.
29. The method according to any one of aspect 1 and embodiments 2 to 28, wherein the antigen is Fibroblast Activation Protein (FAP).
30. The method according to any one of aspect 1 and embodiments 2 to 29, wherein the variable domains of the antibody heavy chain and the antibody light chain form a binding site specifically binding to FAP and comprise a VH domain comprising (i) CDR-H1 comprising the amino acid sequence of SEQ ID NO: 06 or SEQ ID NO: 60, (ii) CDR-H2 comprising the amino acid sequence of SEQ ID NO: 07 or SEQ ID NO: 61, and (iii) CDR-H3 comprising the amino acid sequence of SEQ ID NO: 08 or SEQ ID NO: 62, and a VL domain comprising (iv) CDR-L1 comprising the amino acid sequence of SEQ ID NO: 09 or SEQ ID NO: 63, (v) CDR-L2 comprising the amino acid sequence of SEQ ID NO: 10 or SEQ ID NO: 64, and (vi) CDR-L3 comprising the amino acid sequence of SEQ ID NO: 11 or SEQ ID NO: 65.
31. The method according to any one of aspect 1 and embodiments 2 to 30, wherein the variable domains of the antibody heavy chain and the antibody light chain form a binding site specifically binding to FAP and comprise a variable heavy chain domain comprising an amino acid sequence of SEQ ID NO: 15 and a variable light chain domain comprising an amino acid sequence of SEQ ID NO: 16, or a variable heavy chain domain comprising an amino acid sequence of SEQ ID NO: 66 and a variable light chain domain comprising an amino acid sequence of SEQ ID NO: 67, or wherein the first fusion polypeptide comprises the amino acid sequence of SEQ ID NO: 97 and the second fusion polypeptide comprises the amino acid sequence of SEQ ID NO: 98.
32. The method according to any one of aspect 1 and embodiments 2 to 31, wherein
   (i) the antibody heavy chain comprises a VH domain comprising the amino acid sequence of SEQ ID NO: 15 and the antibody light chain comprises a VL domain comprising the amino acid sequence of SEQ ID NO: 16, or the antibody heavy chain comprises a VH domain comprising the amino acid sequence of SEQ ID NO: 66 and the antibody light chain comprises a VL domain comprising the amino acid sequence of SEQ ID NO: 67,
   (ii) the first fusion polypeptide comprises an amino acid sequence selected from the group consisting of SEQ ID NO: 13, SEQ ID NO: 68, SEQ ID NO: 71 and SEQ ID NO: 73, and the second fusion polypeptide comprises the amino acid sequence of SEQ ID NO: 14, SEQ ID NO: 69, SEQ ID NO: 70, SEQ ID NO: 72 and SEQ ID NO: 74.
33. The method according to any one of aspect 1 and embodiments 2 to 32, wherein
   (i) the antibody heavy chain comprises a VH domain comprising the amino acid sequence of SEQ ID NO: 15 and the antibody light chain comprises a VL domain comprising the amino acid sequence of SEQ ID NO: 16, or the antibody heavy chain comprises a VH domain comprising the amino acid sequence of SEQ ID NO: 66 and the antibody light chain comprises a VL domain comprising the amino acid sequence of SEQ ID NO: 67, (ii) the first fusion polypeptide comprises the amino acid sequence selected from the group consisting of SEQ ID NO: 75, SEQ ID NO: 77, SEQ ID NO: 79 and SEQ ID NO: 82, and the second fusion polypeptide comprises an amino acid sequence selected from the group consisting of SEQ ID NO: 76, SEQ ID NO: 78, SEQ ID NO: 80 and SEQ ID NO: 83.
34. The method according to any one of claims 1 to 28 and 42 to 47, wherein the antibody heavy chain and the antibody light chain form a binding site specifically binding to (human) FAP and the antibody heavy chain has the amino acid sequence of SEQ ID NO: 141 and the light chain has the amino acid sequence of SEQ ID NO: 142, wherein the first fusion polypeptide comprises the amino acid sequence of SEQ ID NO: 79, and the second fusion polypeptide comprises the amino acid sequence of SEQ ID NO: 80.
35. The method according to any one of aspect 1 or embodiments 2 to 28, wherein the antigen is CEA.
36. The method according to any one of aspect 1 and embodiments 2 to 28 and 35, wherein the variable domains of the antibody heavy chain and the antibody light chain form a binding site specifically binding to CEA and comprise a VH domain comprising (i) CDR-H1 comprising the amino acid sequence of SEQ ID NO: 84, (ii) CDR-H2 comprising the amino acid sequence of SEQ ID NO: 85, and (iii) CDR-H3 comprising the amino acid sequence of SEQ ID NO: 86, and a VL domain comprising (iv) CDR-L1 comprising the amino acid sequence of SEQ ID NO: 87, (v) CDR-L2 comprising the amino acid sequence of SEQ ID NO: 88, and (vi) CDR-L3 comprising the amino acid sequence of SEQ ID NO: 89.
37. The method according to any one aspect 1 and embodiments 2 to 28 and 35 to 36, wherein the variable domains of the antibody heavy chain and the antibody light chain form a binding site specifically binding to CEA and comprise a variable heavy chain domain comprising an amino acid sequence of SEQ ID NO: 90 and a variable light chain domain comprising an amino acid sequence of SEQ ID NO: 91.
38. The method according to any one of aspect 1 and embodiments 2 to 28 and 35 to 37, wherein antibody-multimer-fusion comprises
   (i) a heavy chain comprising the VH domain comprising the amino acid sequence of SEQ ID NO: 90 and a light chain comprising the VL domain comprising the amino acid sequence of SEQ ID NO: 91,
   (ii) a first fusion polypeptide comprising the amino acid sequence selected from the group consisting of SEQ ID NO: 13, SEQ ID NO: 68, SEQ ID NO: 71 and SEQ ID NO: 73, and
   (iii) a second fusion polypeptide comprising the amino acid sequence of SEQ ID NO: 14, SEQ ID NO: 69, SEQ ID NO: 70, SEQ ID NO: 72 and SEQ ID NO: 74.
39. The method according to any one of aspect 1 and embodiments 2 to 28 and 35 to 38, wherein antibody-multimer-fusion comprises
   (i) a heavy chain comprising the VH domain comprising the amino acid sequence of SEQ ID NO: 90 and a light chain comprising the VL domain comprising the amino acid sequence of SEQ ID NO: 91,
   (ii) a first fusion polypeptide comprising the amino acid sequence selected from the group consisting of SEQ ID NO: 75, SEQ ID NO: 77, SEQ ID NO: 79 and SEQ ID NO: 82, and
   (iii) a second fusion polypeptide comprising the amino acid sequence selected from the group consisting of SEQ ID NO: 76, SEQ ID NO: 78, SEQ ID NO: 80 and SEQ ID NO: 83.
40. The method according to any one aspect 1 and embodiments 2 to 28 and 35 to 39, wherein antibody-multimer-fusion comprises
   (i) a first heavy chain comprising the amino acid sequence of SEQ ID NO: 93, a second heavy chain comprising the amino acid sequence of SEQ ID NO: 94, and two light chains comprising the amino acid sequence of SEQ ID NO: 92, or
   (ii) a first heavy chain comprising the amino acid sequence of SEQ ID NO: 95, a second heavy chain comprising the amino acid sequence of SEQ ID NO: 96, and two light chains comprising the amino acid sequence of SEQ ID NO: 92.
41. The method according to any one of aspect 1 or embodiments 2 to 28 and 35 to 40, wherein the antibody heavy chain and the antibody light chain form a binding site specifically binding to (human) CEA and the antibody heavy chain has the amino acid sequence of SEQ ID NO: 143 and the light chain has the amino acid sequence of SEQ ID NO: 92, wherein the first fusion polypeptide comprises the amino acid sequence of SEQ ID NO: 79, and the second fusion polypeptide comprises the amino acid sequence of SEQ ID NO: 80.
42. The method according to any one of aspect 1 or embodiments 2 to 28, wherein the antigen is CD19.
43. The method according to any one aspect 1 or embodiments 2 to 28 and 42, wherein the variable domains of the antibody heavy chain and the antibody light chain form a binding site specifically binding to CD19 and comprise a VH domain comprising (i) CDR-H1 comprising the amino acid sequence of SEQ ID NO: 104 or SEQ ID NO: 105, (ii) CDR-H2 comprising the amino acid sequence of SEQ ID NO: 106 or SEQ ID NO: 107, and (iii) CDR-H3 comprising the amino acid sequence of SEQ ID NO: 108 or SEQ ID NO: 109, and a VL domain comprising (iv) CDR-L1 comprising the amino acid sequence of SEQ ID NO: 110 or SEQ ID NO: 111, (v) CDR-L2 comprising the amino acid sequence of SEQ ID NO: 112 or SEQ ID NO: 113, and (vi) CDR-L3 comprising the amino acid sequence of SEQ ID NO: 114 or SEQ ID NO: 115.
44. The method according to any one aspect 1 or embodiments 2 to 28 and 42 to 43, wherein the variable domains of the antibody heavy chain and the antibody light chain form a binding site specifically binding to CD19 and comprise a variable heavy chain domain comprising an amino acid sequence of SEQ ID NO: 116 and a variable light chain domain comprising an amino acid sequence of SEQ ID NO: 117, or comprise a variable heavy chain domain comprising an amino acid sequence of SEQ ID NO: 118 and a variable light chain domain comprising an amino acid sequence of SEQ ID NO: 119.
45. The method according to any one aspect 1 or embodiments 2 to 28 and 42 to 44, wherein
   (i) the heavy chain comprises a VH domain comprising the amino acid sequence of SEQ ID NO: 116 and the light chain comprises a VL domain comprising the amino acid sequence of SEQ ID NO: 117, or the heavy chain comprises a VH domain comprising the amino acid sequence of SEQ ID NO: 118 and the light chain comprises a VL domain comprising the amino acid sequence of SEQ ID NO: 119,
   (ii) the first fusion polypeptide comprises the amino acid sequence selected from the group consisting of SEQ ID NO: 13, SEQ ID NO: 68, SEQ ID NO: 71 and SEQ ID NO: 73, and
   (iii) the second fusion polypeptide comprises the amino acid sequence of SEQ ID NO: 14, SEQ ID NO: 69, SEQ ID NO: 70, SEQ ID NO: 72 and SEQ ID NO: 74.
46. The method according to any one aspect 1 or embodiments 2 to 28 and 42 to 45, wherein
   (i) the heavy chain comprises a VH domain comprising the amino acid sequence of SEQ ID NO: 116 and the light chain comprises a VL domain comprising the amino acid sequence of SEQ ID NO: 117, or the heavy chain comprises a VH domain comprising the amino acid sequence of SEQ ID NO: 118 and the light chain comprises a VL domain comprising the amino acid sequence of SEQ ID NO: 119,
   (ii) the first fusion polypeptide comprises the amino acid sequence selected from the group consisting of SEQ ID NO: 75, SEQ ID NO: 77, SEQ ID NO: 79 and SEQ ID NO: 82, and
   (iii) the second fusion polypeptide comprises the amino acid sequence selected from the group consisting of SEQ ID NO: 76, SEQ ID NO: 78, SEQ ID NO: 80 and SEQ ID NO: 83.
47. The method according to any one of aspect 1 or embodiments 2 to 28 and 42 to 45, wherein
   (i) the heavy chain comprises the amino acid sequence of SEQ ID NO: 120, the first fusion polypeptide comprises the amino acid sequence of SEQ ID NO: 121, and the light chain comprises the amino acid sequence of SEQ ID NO: 122, or
   (ii) the heavy chain comprises the amino acid sequence of SEQ ID NO: 123, the first fusion polypeptide comprises the amino acid sequence of SEQ ID NO: 124, and the light chain comprises the amino acid sequence of SEQ ID NO: 122, or
   (iii) the heavy chain comprises the amino acid sequence of SEQ ID NO: 125, the first fusion polypeptide comprises the amino acid sequence of SEQ ID NO: 126, and the light chain comprises the amino acid sequence of SEQ ID NO: 127, or
   (iv) the heavy chain comprises the amino acid sequence of SEQ ID NO: 128, the first fusion polypeptide comprises the amino acid sequence of SEQ ID NO: 129, and the light chain comprises the amino acid sequence of SEQ ID NO: 127.
48. The method according to any one of aspect 1 or embodiments 2 to 28 and 42 to 47, wherein the antibody heavy chain and the antibody light chain form a binding site specifically binding to (human) CD19 and the antibody heavy chain has the amino acid sequence of SEQ ID NO: 144 and the light chain has the amino acid sequence of SEQ ID NO: 127, wherein the first fusion polypeptide comprises the amino acid sequence of SEQ ID NO: 79, and the second fusion polypeptide comprises the amino acid sequence of SEQ ID NO: 80.
49. The method according to any one of aspect 1 or embodiments 2 to 48, wherein the transfecting a (parent) mammalian cell is a targeted integration transfecting.
50. The method according to embodiment 49, wherein the targeted integration transfecting is a double recombinase mediated cassette exchange.
51. The method according to any one of embodiments 49 to 50, wherein the (parent) mammalian cell is a CHO cell harboring a landing site integrated at a single site within a locus of its genome.
52. The method according to embodiment 51, wherein the landing site comprises a first and a second selection marker, which are flanked by two RRSs, wherein the first selection marker is different from the second selection marker.
53. The method according to embodiment 52, wherein the first selection marker is a glutamine synthetase selection marker and the second selection marker is a GFP fluorescent protein.
54. The method according to embodiment 52, wherein the integrated landing site comprises a thymidine kinase selection marker and a HYG selection marker.
55. The method according to any one of embodiments 52 to 54, wherein the two RRSs flanking both selection markers are different.
56. The method according to any one of embodiments 51 to 55, wherein the landing site comprises three heterospecific loxP sites for Cre recombinase mediated DNA recombination.
57. The method according to embodiment 56, wherein the heterospecific loxP sites are L3, LoxFas and 2L.
58. The method according to embodiment 57, wherein the L3 and 2L flank the landing site at the 5'-end and 3'-end, respectively, and LoxFas is located between the L3 and 2L sites.
59. The method according to any one of embodiments 51 to 58, wherein the landing site further contains a bicistronic unit linking the expression of a selection marker via an IRES to the expression of the fluorescent GFP protein.
60. The method according to any one of aspect 1 or embodiments 2 to 59, wherein the antibody-multimer-fusion polypeptide is expressed from a deoxyribonucleic acid integrated in the genome of the cell that comprises in 5'- to 3'-direction
   - a first expression cassette encoding the first fusion polypeptide,
   - a second expression cassette encoding the first fusion polypeptide,
   - a third expression cassette encoding the second fusion polypeptide,
   - a fourth expression cassette encoding the antibody heavy chain, and
   - a fifth expression cassette encoding the antibody light chain.
61. The method according to any one of aspect 1 or embodiments 2 to 60, wherein the deoxyribonucleic acid encoding the antibody-multimer-fusion polypeptide is stably integrated into the genome of the mammalian cell at a single site or locus.
62. The method according to any one of embodiments 60 to 61, wherein the deoxyribonucleic acid encoding the antibody-multimer-fusion polypeptide further comprises
   - a first recombination recognition sequence located 5' to the first (most 5') expression cassette,
   - a second recombination recognition sequence located 3' to the fifth (most 3') expression cassette, and
   - a third recombination recognition sequence located
   - between the first and the second recombination recognition sequence, and
   - between the third and the fourth expression cassette,
   and
   wherein all recombination recognition sequences are different.
63. The method according to any one of embodiments 60 to 62, wherein the deoxyribonucleic acid encoding the antibody-multimer-fusion polypeptide further comprises a further expression cassette encoding for a selection marker.
64. The method according to embodiment 63, wherein the expression cassette encoding for a selection marker is located either
   i) 5', or
   ii) 3', or
   iii) partly 5' and partly 3'
   to the third recombination recognition sequence.
65. The method according to any one of embodiments 63 to 64, wherein the expression cassette encoding for a selection marker is located partly 5' and partly 3' to the third recombination recognition sequences, wherein the 5'-located part of said expression cassette comprises the promoter and a start-codon and the 3'-located part of said expression cassette comprises the coding sequence without a start-codon and a polyA signal.
66. The method according to any one of embodiments 60 to 65, wherein the deoxyribonucleic acid encoding the antibody-multimer-fusion polypeptide comprises a further expression cassette encoding for a selection marker and the expression cassette encoding for the selection marker is located partly 5' and partly 3' to the third recombination recognition sequence, wherein the 5'-located part of said expression cassette comprises the promoter and the start-codon and the 3'-located part of said expression cassette comprises the coding sequence without a start-codon and a polyA signal, wherein the start-codon is operably linked to the coding sequence.
67. The method according to any one of embodiments 65 to 66, wherein the start-codon is ATG.

### Detailed Description of Embodiments of the Invention

The current invention is based, at least in part, on the finding that for the expression of an antibody-multimer-fusion, which is a complex molecule comprising different polypeptides, i.e. which is a heteromultimer, the use of a defined expression cassette ratio results in efficient expression and production of the antibody-multimer-fusion in mammalian cells, such as HEK or CHO cells.

The current invention is based, at least in part, on the finding that for transient as well as stable expression of an antibody-multimer-fusion, which is a complex molecule comprising different polypeptides, i.e. which is a heteromultimer, the use of the same defined expression cassette ratio results in the highest expression yield and product quality.

### I. DEFINITIONS

As used herein, the amino acid positions of all constant regions and domains of the heavy and light chain are numbered according to the Kabat numbering system described in Kabat, et al., Sequences of Proteins of Immunological Interest, 5th ed., Public Health Service, National Institutes of Health, Bethesda, MD (1991) and is referred to as "numbering according to Kabat" herein. Specifically, the Kabat numbering system (see pages 647-660) of Kabat, et al., Sequences of Proteins of Immunological Interest, 5th ed., Public Health Service, National Institutes of Health, Bethesda, MD (1991) is used for the light chain constant domain CL of kappa and lambda isotype, and the Kabat EU index numbering system (see pages 661-723) is used for the constant heavy chain domains (CH1, Hinge, CH2 and CH3, which is herein further clarified by referring to "numbering according to Kabat EU index" in this case).

The knobs into holes dimerization modules and their use in antibody engineering are described in Carter P.; Ridgway J.B.B.; Presta L.G.: Immunotechnology 2 (1996) 73-73(1).

General information regarding the nucleotide sequences of human immunoglobulins light and heavy chains is given in: Kabat, E.A., et al., Sequences of Proteins of Immunological Interest, 5th ed., Public Health Service, National Institutes of Health, Bethesda, MD (1991).

Useful methods and techniques for carrying out the current invention are described in e.g. Ausubel, F.M. (ed.), Current Protocols in Molecular Biology, Volumes I to III (1997); Glover, N.D., and Hames, B.D., ed., DNA Cloning: A Practical Approach, Volumes I and II (1985), Oxford University Press; Freshney, R.I. (ed.), Animal Cell Culture - a practical approach, IRL Press Limited (1986); Watson, J.D., et al., Recombinant DNA, Second Edition, CHSL Press (1992); Winnacker, E.L., From Genes to Clones; N.Y., VCH Publishers (1987); Celis, J., ed., Cell Biology, Second Edition, Academic Press (1998); Freshney, R.I., Culture of Animal Cells: A Manual of Basic Technique, second edition, Alan R. Liss, Inc., N.Y. (1987).

The use of recombinant DNA technology enables the generation of derivatives of a nucleic acid. Such derivatives can, for example, be modified in individual or several nucleotide positions by substitution, alteration, exchange, deletion or insertion. The modification or derivatization can, for example, be carried out by means of site directed mutagenesis. Such modifications can easily be carried out by a person skilled in the art (see e.g. Sambrook, J., et al., Molecular Cloning: A laboratory manual (1999) Cold Spring Harbor Laboratory Press, New York, USA; Hames, B.D., and Higgins, S.G., Nucleic acid hybridization - a practical approach (1985) IRL Press, Oxford, England).

It must be noted that as used herein and in the appended embodiments, the singular forms "a", "an", and "the" include plural reference unless the context clearly dictates otherwise. Thus, for example, reference to "a cell" includes a plurality of such cells and equivalents thereof known to those skilled in the art, and so forth. As well, the terms "a" (or "an"), "one or more" and "at least one" can be used interchangeably herein. It is also to be noted that the terms "comprising", "including", and "having" can be used interchangeably.

The term "about" denotes a range of +/- 20 % of the thereafter following numerical value. In one embodiment the term about denotes a range of +/- 10 % of the thereafter following numerical value. In one embodiment the term about denotes a range of +/-5 % of the thereafter following numerical value.

The term "comprising" also encompasses the term "consisting of'.

The term "recombinant cell" as used herein denotes a cell after final genetic modification, such as, e.g., a cell expressing a polypeptide of interest and that can be used for the production of said polypeptide of interest at any scale. For example, "a mammalian cell comprising an exogenous nucleotide sequence" that has been subjected to recombinase mediated cassette exchange (RMCE) whereby the coding sequences for a polypeptide of interest have been introduced into the genome of the host cell is a "recombinant cell". Although the cell is still capable of performing further RMCE reactions, it is not intended to do so.

The term "recombinant mammalian cell" as used herein denotes a mammalian cell comprising an exogenous nucleotide sequence capable of expressing a polypeptide. Such recombinant mammalian cells are cells into which one or more exogenous nucleic acid(s) have been introduced, including the progeny of such cells. Thus, the term "a mammalian cell comprising a nucleic acid encoding a heterologous polypeptide" denotes cells comprising an exogenous nucleotide sequence integrated in the genome of the mammalian cell and capable of expressing the heterologous polypeptide. In one embodiment the mammalian cell comprising an exogenous nucleotide sequence is a cell comprising an exogenous nucleotide sequence integrated at a single site within a locus of the genome of the host cell, wherein the exogenous nucleotide sequence comprises a first and a second recombination recognition sequence flanking at least one first selection marker, and a third recombination recognition sequence located between the first and the second recombination recognition sequence, and all the recombination recognition sequences are different

A "mammalian cell comprising an exogenous nucleotide sequence" and a "recombinant cell" are both "transformed cells". This term includes the primary transformed cell as well as progeny derived therefrom without regard to the number of passages. Progeny may, e.g., not be completely identical in nucleic acid content to a parent cell, but may contain mutations. Mutant progeny that has the same function or biological activity as screened or selected for in the originally transformed cell are encompassed.

The term "integration site" denotes a nucleic acid sequence within a cell's genome into which an exogenous nucleotide sequence is inserted. In certain embodiments, an integration site is between two adjacent nucleotides in the cell's genome. In certain embodiments, an integration site includes a stretch of nucleotide sequences. In certain embodiments, the integration site is located within a specific locus of the genome of a mammalian cell. In certain embodiments, the integration site is within an endogenous gene of a mammalian cell.

The terms "vector" or "plasmid", which can be used interchangeably, as used herein, refer to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors".

The term "binding to" denotes the binding of a binding site to its target, such as e.g. of an antibody binding site comprising an antibody heavy chain variable domain and an antibody light chain variable domain to the respective antigen. This binding can be determined using, for example, a BIAcore^{®} assay (GE Healthcare, Uppsala, Sweden). That is, the term "binding (to an antigen)" denotes the binding of an antibody in an in vitro assay to its antigen(s). In one embodiment binding is determined in a binding assay in which the antibody is bound to a surface and binding of the antigen to the antibody is measured by Surface Plasmon Resonance (SPR). Binding means e.g. a binding affinity (K_{D}) of 10⁻⁸ M or less, in some embodiments of 10⁻¹³ to 10⁻⁸ M, in some embodiments of 10⁻¹³ to 10⁻⁹ M. The term "binding" also includes the term "specifically binding".

For example, in one possible embodiment of the BIAcore^{®} assay, the antigen is bound to a surface and binding of the antibody, i.e. its binding site(s), is measured by surface plasmon resonance (SPR). The affinity of the binding is defined by the terms kₐ (association constant: rate constant for the association to form a complex), k_{d} (dissociation constant; rate constant for the dissociation of the complex), and K_{D} (k_{d}/kₐ). Alternatively, the binding signal of a SPR sensorgram can be compared directly to the response signal of a reference, with respect to the resonance signal height and the dissociation behaviors.

The term "binding site" denotes any proteinaceous entity that shows binding specificity to a target. This can be, e.g., a receptor, a receptor ligand, an anticalin, an affibody, an antibody, etc. Thus, the term "binding site" as used herein denotes a polypeptide that can specifically bind to or can be specifically bound by a second polypeptide.

As used herein, the term "exogenous" indicates that a nucleotide sequence does not originate from a specific cell and is introduced into said cell by DNA delivery methods, e.g., by transfection, electroporation, or transformation methods. Thus, an exogenous nucleotide sequence is an artificial sequence wherein the artificiality can originate, e.g., from the combination of subsequences of different origin (e.g. a combination of a recombinase recognition sequence with an SV40 promoter and a coding sequence of green fluorescent protein is an artificial nucleic acid) or from the deletion of parts of a sequence (e.g. a sequence coding only the extracellular domain of a membrane-bound receptor or a cDNA) or the mutation of nucleobases. The term "endogenous" refers to a nucleotide sequence originating from a cell. An "exogenous" nucleotide sequence can have an "endogenous" counterpart that is identical in base compositions, but where the "exogenous" sequence is introduced into the cell, e.g., via recombinant DNA technology.

As used herein, the term "selection marker" denotes a gene that allows cells carrying the gene to be specifically selected for or against, in the presence of a corresponding selection agent. For example, but not by way of limitation, a selection marker can allow the host cell transformed with the selection marker gene to be positively selected for in the presence of the respective selection agent (selective cultivation conditions); a non-transformed host cell would not be capable of growing or surviving under the selective cultivation conditions. Selection markers can be positive, negative or bi-functional. Positive selection markers can allow selection for cells carrying the marker, whereas negative selection markers can allow cells carrying the marker to be selectively eliminated. A selection marker can confer resistance to a drug or compensate for a metabolic or catabolic defect in the host cell. In prokaryotic cells, amongst others, genes conferring resistance against ampicillin, tetracycline, kanamycin or chloramphenicol can be used. Resistance genes useful as selection markers in eukaryotic cells include, but are not limited to, genes for aminoglycoside phosphotransferase (APH) (e.g., hygromycin phosphotransferase (HYG), neomycin and G418 APH), dihydrofolate reductase (DHFR), thymidine kinase (TK), glutamine synthetase (GS), asparagine synthetase, tryptophan synthetase (indole), histidinol dehydrogenase (histidinol D), and genes encoding resistance to puromycin, blasticidin, bleomycin, phleomycin, chloramphenicol, Zeocin, and mycophenolic acid. Further marker genes are described in WO 92/08796 and WO 94/28143.

Beyond facilitating a selection in the presence of a corresponding selection agent, a selection marker can alternatively be a molecule normally not present in the cell, e.g., green fluorescent protein (GFP), enhanced GFP (eGFP), synthetic GFP, yellow fluorescent protein (YFP), enhanced YFP (eYFP), cyan fluorescent protein (CFP), mPlum, mCherry, tdTomato, mStrawberry, J-red, DsRed-monomer, mOrange, mKO, mCitrine, Venus, YPet, Emerald, CyPet, mCFPm, Cerulean, and T-Sapphire. Cells expressing such a molecule can be distinguished from cells not harboring this gene, e.g., by the detection or absence, respectively, of the fluorescence emitted by the encoded polypeptide.

The term "Fibroblast activation protein (FAP)", also known as Prolyl endopeptidase FAP or Seprase (EC 3.4.21), refers to any native FAP from any vertebrate source, including mammals such as primates (e.g. humans) non-human primates (e.g. cynomolgus monkeys) and rodents (e.g. mice and rats), unless otherwise indicated. The term encompasses "full-length," unprocessed FAP as well as any form of FAP which results from processing in the cell. The term also encompasses naturally occurring variants of FAP, e.g., splice variants or allelic variants. In one embodiment, the antigen binding molecule of the invention is capable of specific binding to human, mouse and/or cynomolgus FAP. The amino acid sequence of human FAP is shown in UniProt (www.uniprot.org) accession no. Q12884 (version 149, SEQ ID NO: 17), or NCBI (www.ncbi.nlm.nih.gov/) RefSeqNP_004451.2. The extracellular domain (ECD) of human FAP extends from amino acid position 26 to 760. The amino acid and nucleotide sequences of a His-tagged human FAP ECD is shown in SEQ ID NO: 14 and SEQ ID NO: 15, respectively. The amino acid sequence of mouse FAP is shown in UniProt accession no. P97321 (version 126, SEQ ID NO: 18), or NCBI RefSeq NP_032012.1. The extracellular domain (ECD) of mouse FAP extends from amino acid position 26 to 761. SEQ ID NO: 19 and SEQ ID NO: 20 show the amino acid and nucleotide sequences, respectively, of a His-tagged mouse FAP ECD. SEQ ID NO: 21 and SEQ ID NO: 22 show the amino acid and nucleotide sequences, respectively, of a His-tagged cynomolgus FAP ECD. Preferably, an anti-FAP binding molecule of the invention binds to the extracellular domain of FAP. Exemplary anti-FAP binding molecules are described in WO 2012/020006.

The term "TNF ligand family member" or "TNF family ligand" refers to a pro-inflammatory cytokine. Cytokines in general, and in particular the members of the TNF ligand family, play a crucial role in the stimulation and coordination of the immune system. At present, nineteen cytokines have been identified as members of the TNF (tumor necrosis factor) ligand superfamily on the basis of sequence, functional, and structural similarities. All these ligands are type II transmembrane proteins with a C-terminal extracellular domain (ectodomain), N-terminal intracellular domain and a single transmembrane domain. The C-terminal extracellular domain, known as TNF homology domain (THD), has 20-30% amino acid identity between the superfamily members and is responsible for binding to the receptor. The TNF ectodomain is also responsible for the TNF ligands to form trimeric complexes that are recognized by their specific receptors.

Members of the TNF ligand family are selected from the group consisting of Lymphotoxin α (also known as LTA or TNFSF1), TNF (also known as TNFSF2), LTβ (also known as TNFSF3), OX40L (also known as TNFSF4), CD40L (also known as CD154 or TNFSF5), FasL (also known as CD95L, CD178 or TNFSF6), CD27L (also known as CD70 or TNFSF7), CD30L (also known as CD153 or TNFSF8), 4-1-BBL (also known as TNFSF9), TRAIL (also known as APO2L, CD253 or TNFSF10), RANKL (also known as CD254 or TNFSF11), TWEAK (also known as TNFSF12), APRIL (also known as CD256 or TNFSF13), BAFF (also known as CD257 or TNFSF13B), LIGHT (also known as CD258 or TNFSF14), TL1A (also known as VEGI or TNFSF15), GITRL (also known as TNFSF18), EDA-A1 (also known as ectodysplasin A1) and EDA-A2 (also known as ectodysplasin A2). The term refers to any native TNF family ligand from any vertebrate source, including mammals such as primates (e.g. humans), non-human primates (e.g. cynomolgus monkeys) and rodents (e.g. mice and rats), unless otherwise indicated. In specific embodiments of the invention, the TNF ligand family member is selected from the group consisting of OX40L, FasL, CD27L, TRAIL, 4-1-BBL, CD40L and GITRL. In a particular embodiment, the TNF ligand family member is selected from 4-1-BBL and OX40L.

Further information, in particular sequences, of the TNF ligand family members may be obtained from publically accessible databases such as UniProt (www.uniprot.org). For instance, the human TNF ligands have the following amino acid sequences: human Lymphotoxin α (UniProt accession no. P01374, SEQ ID NO: 24), human TNF (UniProt accession no. P01375, SEQ ID NO: 25), human Lymphotoxin α (UniProt accession no. Q06643, SEQ ID NO: 26), human OX40L (UniProt accession no. P23510, SEQ ID NO: 27), human CD40L (UniProt accession no. P29965, SEQ ID NO: 28), human FasL (UniProt accession no. P48023, SEQ ID NO: 29), human CD27L (UniProt accession no. P32970, SEQ ID NO: 30), human CD30L (UniProt accession no. P32971, SEQ ID NO: 31), 4-1-BBL (UniProt accession no. P41273, SEQ ID NO: 32), TRAIL (UniProt accession no. P50591, SEQ ID NO: 33), RANKL (UniProt accession no. 014788, SEQ ID NO: 34), TWEAK (UniProt accession no. O43508, SEQ ID NO: 35), APRIL (UniProt accession no. O75888, SEQ ID NO: 36), BAFF (UniProt accession no. Q9Y275, SEQ ID NO: 37), LIGHT (UniProt accession no. O43557, SEQ ID NO: 38), TL1A (UniProt accession no. 095150, SEQ ID NO: 39), GITRL (UniProt accession no. Q9UNG2, SEQ ID NO: 40) and ectodysplasin A (UniProt accession no. Q92838, SEQ ID NO: 41).

An "ectodomain" is the domain of a membrane protein that extends into the extracellular space (i.e. the space outside the target cell). Ectodomains are usually the parts of proteins that initiate contact with surfaces, which leads to signal transduction. The ectodomain of TNF ligand family member as defined herein thus refers to the part of the TNF ligand protein that extends into the extracellular space (the extracellular domain), but also includes shorter parts or fragments thereof that are responsible for the trimerization and for the binding to the corresponding TNF receptor. The term "ectodomain of a TNF ligand family member or a fragment thereof' thus refers to the extracellular domain of the TNF ligand family member that forms the extracellular domain or to parts thereof that are still able to bind to the receptor (receptor binding domain).

The term "costimulatory TNF ligand family member" or "costimulatory TNF family ligand" refers to a subgroup of TNF ligand family members, which are able to co-stimulate proliferation and cytokine production of T-cells. These TNF family ligands can co-stimulate TCR signals upon interaction with their corresponding TNF receptors and the interaction with their receptors leads to recruitment of TNFR-associated factors (TRAF), which initiate signaling cascades that result in T-cell activation. Costimulatory TNF family ligands are selected from the group consisting of 4-1-BBL, OX40L, GITRL, CD70, CD30L and LIGHT, more particularly the costimulatory TNF ligand family member is selected from 4-1-BBL and OX40L.

As described herein before, 4-1-BBL is a type II transmembrane protein and one member of the TNF ligand family. Complete or full-length 4-1-BBL having the amino acid sequence of SEQ ID NO: 32 has been described to form trimers on the surface of cells. The formation of trimers is enabled by specific motives of the ectodomain of 4-1-BBL. Said motives are designated herein as "trimerization region". The amino acids 50-254 of the human 4-1-BBL sequence (SEQ ID NO: 42) form the extracellular domain of 4-1-BBL, but even fragments thereof are able to form the trimers. In specific embodiments of the invention, the term "ectodomain of 4-1-BBL or a fragment thereof' refers to a polypeptide having an amino acid sequence selected from SEQ ID NO: 04 (amino acids 52-254 of human 4-1-BBL), SEQ ID NO: 01 (amino acids 71-254 of human 4-1-BBL), SEQ ID NO: 03 (amino acids 80-254 of human 4-1-BBL) and SEQ ID NO: 02 (amino acids 85-254 of human 4-1-BBL) or a polypeptide having an amino acid sequence selected from SEQ ID NO: 56 (amino acids 71-248 of human 4-1-BBL), SEQ ID NO: 102 (amino acids 52-248 of human 4-1-BBL), SEQ ID NO: 101 (amino acids 80-248 of human 4-1-BBL) and SEQ ID NO: 100 (amino acids 85-248 of human 4-1-BBL), but also other fragments of the ectodomain capable of trimerization are included herein.

As described herein before, OX40L is another type II transmembrane protein and a further member of the TNF ligand family. Complete or full length human OX40L has the amino acid sequence of SEQ ID NO: 27. The amino acids 51-183 of the human OX40L sequence (SEQ ID NO: 43) form the extracellular domain of OX40L, but even fragments thereof that are able to form the trimers. In specific embodiments of the invention, the term "ectodomain of OX40L or a fragment thereof' refers to a polypeptide having an amino acid sequence selected from SEQ ID NO: 43 (amino acids 51-183 of human OX40L) or SEQ ID NO: 44 (amino acids 52-183 of human OX40L), but also other fragments of the ectodomain capable of trimerization are included herein.

The term "peptide linker" refers to a peptide comprising one or more amino acids, typically about 2 to 20 amino acids. Peptide linkers are known in the art or are described herein. Suitable, non-immunogenic linker peptides are, for example, (G4S)n, (SG4)n or G4(SG4)n peptide linkers, wherein "n" is generally a number between 1 and 10, typically between 1 and 4, in particular 2, i.e. the peptides selected from the group consisting of GGGGS (SEQ ID NO: 81), GGGGSGGGGS (SEQ ID NO: 12), SGGGGSGGGG (SEQ ID NO: 45) and GGGGSGGGGSGGGG (SEQ ID NO: 46), but also include the sequences GSPGSSSSGS (SEQ ID NO: 47), GSGSGSGS (SEQ ID NO: 48), GSGSGNGS (SEQ ID NO: 49), GGSGSGSG (SEQ ID NO: 50), GGSGSG (SEQ ID NO: 51), GGSG (SEQ ID NO: 52), GGSGNGSG (SEQ ID NO: 53), GGNGSGSG (SEQ ID NO: 54) and GGNGSG (SEQ ID NO: 55). Peptide linkers of particular interest are (G4S)1 or GGGGS (SEQ ID NO: 81), (G4S)2 or GGGGSGGGGS (SEQ ID NO: 12) and GSPGSSSSGS (SEQ ID NO: 47), more particularly (G4S)2 or GGGGSGGGGS (SEQ ID NO: 12) and GSPGSSSSGS (SEQ ID NO: 47).

By "fused" or "connected" is meant that the components (e.g. a polypeptide and an ectodomain of said TNF ligand family member) are linked by peptide bonds, either directly or via one or more peptide linkers.

General information regarding the nucleotide sequences of human immunoglobulins light and heavy chains is given in: Kabat, E.A., et al., Sequences of Proteins of Immunological Interest, 5th ed., Public Health Service, National Institutes of Health, Bethesda, MD (1991).

The term "heavy chain" is used herein with its original meaning, i.e. denoting the two larger polypeptide chains of the four polypeptide chains forming an antibody (see, e.g., Edelman, G.M. and Gally J.A., J. Exp. Med. 116 (1962) 207-227). The term "larger" in this context can refer to any of molecular weight, length and amino acid number. The term "heavy chain" is independent from the sequence and number of individual antibody domains present therein. It is solely assigned based on the molecular weight of the respective polypeptide.

The term "light chain" is used herein with its original meaning, i.e. denoting the smaller polypeptide chains of the four polypeptide chains forming an antibody (see, e.g., Edelman, G.M. and Gally J.A., J. Exp. Med. 116 (1962) 207-227). The term "smaller" in this context can refer to any of molecular weight, length and amino acid number. The term "light chain" is independent from the sequence and number of individual antibody domains present therein. It is solely assigned based on the molecular weight of the respective polypeptide.

As used herein, the amino acid positions of all constant regions and domains of the heavy and light chain are numbered according to the Kabat numbering system described in Kabat, et al., Sequences of Proteins of Immunological Interest, 5th ed., Public Health Service, National Institutes of Health, Bethesda, MD (1991) and is referred to as "numbering according to Kabat" herein. Specifically, the Kabat numbering system (see pages 647-660) of Kabat, et al., Sequences of Proteins of Immunological Interest, 5th ed., Public Health Service, National Institutes of Health, Bethesda, MD (1991) is used for the light chain constant domain CL of kappa and lambda isotype, and the Kabat EU index numbering system (see pages 661-723) of Kabat, et al., Sequences of Proteins of Immunological Interest, 5th ed., Public Health Service, National Institutes of Health, Bethesda, MD (1991) is used for the constant heavy chain domains (CH1, hinge, CH2 and CH3, which is herein further clarified by referring to "numbering according to Kabat EU index" in this case).

The term "antibody" herein is used in the broadest sense and encompasses various antibody structures, including but not limited to full length antibodies, monoclonal antibodies, multispecific antibodies (e.g., bispecific antibodies), and antibody-antibody fragment-fusions as well as combinations thereof.

The term "antibody binding site" denotes a pair of a heavy chain variable domain and a light chain variable domain. To ensure proper binding to the antigen these variable domains are cognate variable domains, i.e. belong together. An antibody the binding site comprises at least three HVRs (e.g. in case of a VHH) or three-six HVRs (e.g. in case of a naturally occurring, i.e. conventional, antibody with a VH/VL pair). Generally, the amino acid residues of an antibody that are responsible for antigen binding are forming the binding site. These residues are normally contained in a pair of an antibody heavy chain variable domain and a corresponding antibody light chain variable domain. The antigen-binding site of an antibody comprises amino acid residues from the "hypervariable regions" or "HVRs". "Framework" or "FR" regions are those variable domain regions other than the hypervariable region residues as herein defined. Therefore, the light and heavy chain variable domains of an antibody comprise from N- to C-terminus the regions FR1, HVR1, FR2, HVR2, FR3, HVR3 and FR4. Especially, the HVR3 region of the heavy chain variable domain is the region, which contributes most to antigen binding and defines the binding specificity of an antibody. A "functional binding site" is capable of specifically binding to its target. The term "specifically binding to" denotes the binding of a binding site to its target in an in vitro assay, in one embodiment in a binding assay. Such binding assay can be any assay as long the binding event can be detected. For example, an assay in which the antibody is bound to a surface and binding of the antigen(s) to the antibody is measured by Surface Plasmon Resonance (SPR). Alternatively, a bridging ELISA can be used.

The term "hypervariable region" or "HVR", as used herein, refers to each of the regions of an antibody variable domain comprising the amino acid residue stretches which are hypervariable in sequence ("complementarity determining regions" or "CDRs") and/or form structurally defined loops ("hypervariable loops"), and/or contain the antigen-contacting residues ("antigen contacts"). Generally, antibodies comprise six HVRs; three in the heavy chain variable domain VH (H1, H2, H3), and three in the light chain variable domain VL (L1, L2, L3).

### HVRs include

(a) hypervariable loops occurring at amino acid residues 26-32 (L1), 50-52 (L2), 91-96 (L3), 26-32 (H1), 53-55 (H2), and 96-101 (H3) (Chothia, C. and Lesk, A.M., J. Mol. Biol. 196 (1987) 901-917);
(b) CDRs occurring at amino acid residues 24-34 (L1), 50-56 (L2), 89-97 (L3), 31-35b (H1), 50-65 (H2), and 95-102 (H3) (Kabat, E.A. et al., Sequences of Proteins of Immunological Interest, 5th ed. Public Health Service, National Institutes of Health, Bethesda, MD (1991), NIH Publication 91-3242.);
(c) antigen contacts occurring at amino acid residues 27c-36 (L1), 46-55 (L2), 89-96 (L3), 30-35b (H1), 47-58 (H2), and 93-101 (H3) (MacCallum et al. J. Mol. Biol. 262: 732-745 (1996)); and
(d) combinations of (a), (b), and/or (c), including amino acid residues 46-56 (L2), 47-56 (L2), 48-56 (L2), 49-56 (L2), 26-35 (H1), 26-35b (H1), 49-65 (H2), 93-102 (H3), and 94-102 (H3).

Unless otherwise indicated, HVR residues and other residues in the variable domain (e.g., FR residues) are numbered herein according to Kabat et al., *supra.*

The "class" of an antibody refers to the type of constant domains or constant region, preferably the Fc-region, possessed by its heavy chains. There are five major classes of antibodies: IgA, IgD, IgE, IgG, and IgM, and several of these may be further divided into subclasses (isotypes), e.g., IgG1, IgG2, IgG3, IgG4, IgA1, and IgA2. The heavy chain constant domains that correspond to the different classes of immunoglobulins are called α, δ, ε, γ, and µ, respectively.

The term "heavy chain constant region" denotes the region of an immunoglobulin heavy chain that contains the constant domains, i.e. for a native immunoglobulin the CH1 domain, the hinge region, the CH2 domain and the CH3 domain or for a full length immunoglobulin the first constant domain, the hinge region, the second constant domain and the third constant domain. In one embodiment, a human IgG heavy chain constant region extends from Ala118 to the carboxyl-terminus of the heavy chain (numbering according to Kabat EU index). However, the C-terminal lysine (Lys447) of the constant region may or may not be present (numbering according to Kabat EU index). The term "constant region" denotes a dimer comprising two heavy chain constant regions, which can be covalently linked to each other via the hinge region cysteine residues forming inter-chain disulfide bonds.

The term "heavy chain Fc-region" denotes the C-terminal region of an immunoglobulin heavy chain that contains at least a part of the hinge region (middle and lower hinge region), the second constant domain, e.g. the CH2 domain, and the third constant domain, e.g. the CH3 domain. In one embodiment, a human IgG heavy chain Fc-region extends from Asp221, or from Cys226, or from Pro230, to the carboxyl-terminus of the heavy chain (numbering according to Kabat EU index). Thus, an Fc-region is smaller than a constant region but in the C-terminal part identical thereto. However, the C-terminal lysine (Lys447) of the heavy chain Fc-region may or may not be present (numbering according to Kabat EU index). The term "Fc-region" denotes a dimer comprising two heavy chain Fc-regions, which can be covalently linked to each other via the hinge region cysteine residues forming inter-chain disulfide bonds.

The constant region, more precisely the Fc-region, of an antibody (and the constant region likewise) is directly involved in complement activation, C1q binding, C3 activation and Fc receptor binding. While the influence of an antibody on the complement system is dependent on certain conditions, binding to C1q is caused by defined binding sites in the Fc-region. Such binding sites are known in the state of the art and described e.g. by Lukas, T.J., et al., J. Immunol. 127 (1981) 2555-2560; Brunhouse, R., and Cebra, J.J., Mol. Immunol. 16 (1979) 907-917; Burton, D.R., et al., Nature 288 (1980) 338-344; Thommesen, J.E., et al., Mol. Immunol. 37 (2000) 995-1004; Idusogie, E.E., et al., J. Immunol. 164 (2000) 4178-4184; Hezareh, M., et al., J. Virol. 75 (2001) 12161-12168; Morgan, A., et al., Immunology 86 (1995) 319-324; and EP 0 307 434. Such binding sites are e.g. L234, L235, D270, N297, E318, K320, K322, P331 and P329 (numbering according to EU index of Kabat). Antibodies of subclass IgG1, IgG2 and IgG3 usually show complement activation, C1q binding and C3 activation, whereas IgG4 do not activate the complement system, do not bind C1q and do not activate C3. An "Fc-region of an antibody" is a term well known to the skilled artisan and defined on the basis of papain cleavage of antibodies.

The term "monoclonal antibody" as used herein refers to an antibody obtained from a population of substantially homogeneous antibodies, i.e., the individual antibodies comprising the population are identical and/or bind the same epitope, except for possible variant antibodies, e.g., containing naturally occurring mutations or arising during production of a monoclonal antibody preparation, such variants generally being present in minor amounts. In contrast to polyclonal antibody preparations, which typically include different antibodies directed against different determinants (epitopes), each monoclonal antibody of a monoclonal antibody preparation is directed against a single determinant on an antigen. Thus, the modifier "monoclonal" indicates the character of the antibody as being obtained from a substantially homogeneous population of antibodies, and is not to be construed as requiring production of the antibody by any particular method. For example, monoclonal antibodies may be made by a variety of techniques, including but not limited to the hybridoma method, recombinant DNA methods, phage-display methods, and methods utilizing transgenic animals containing all or part of the human immunoglobulin loci.

The term "valent" as used within the current application denotes the presence of a specified number of binding sites in an antibody. As such, the terms "bivalent", "tetravalent", and "hexavalent" denote the presence of two binding site, four binding sites, and six binding sites, respectively, in an antibody.

A "monospecific antibody" denotes an antibody that has a single binding specificity, i.e. specifically binds to one antigen. Monospecific antibodies can be prepared as full-length antibodies or antibody fragments (e.g. F(ab')₂) or combinations thereof (e.g. full length antibody plus additional scFv or Fab fragments). A monospecific antibody does not need to be monovalent, i.e. a monospecific antibody may comprise more than one binding site specifically binding to the one antigen. A native antibody, for example, is monospecific but bivalent.

The "knobs into holes" dimerization modules and their use in antibody engineering are described in Carter P.; Ridgway J.B.B.; Presta L.G.: Immunotechnology 2 (1996) 73-73(1).

The CH3 domains in the heavy chains of an antibody can be altered by the "knob-into-holes" technology, which is described in detail with several examples in e.g. WO 96/027011, Ridgway, J.B., et al., Protein Eng. 9 (1996) 617-621; and Merchant, A.M., et al., Nat. Biotechnol. 16 (1998) 677-681. In this method, the interaction surfaces of the two CH3 domains are altered to increase the heterodimerization of these two CH3 domains and thereby of the polypeptide comprising them. Each of the two CH3 domains (of the two heavy chains) can be the "knob", while the other is the "hole". The introduction of a disulfide bridge further stabilizes the heterodimers (Merchant, A.M., et al., Nature Biotech. 16 (1998) 677-681; Atwell, S., et al., J. Mol. Biol. 270 (1997) 26-35) and increases the yield.

The mutation T366W in the CH3 domain (of an antibody heavy chain) is denoted as "knob-mutation" or "mutation knob" and the mutations T366S, L368A, Y407V in the CH3 domain (of an antibody heavy chain) are denoted as "hole-mutations" or "mutations hole" (numbering according to Kabat EU index). An additional inter-chain disulfide bridge between the CH3 domains can also be used (Merchant, A.M., et al., Nature Biotech. 16 (1998) 677-681) e.g. by introducing a S354C mutation into the CH3 domain of the heavy chain with the "knob-mutation" (denotes as "knob-cys-mutations" or "mutations knob-cys") and by introducing a Y349C mutation into the CH3 domain of the heavy chain with the "hole-mutations" (denotes as "hole-cys-mutations" or "mutations hole-cys") (numbering according to Kabat EU index).

The term "domain crossover" as used herein denotes that in a pair of an antibody heavy chain VH-CH1 fragment and its corresponding cognate antibody light chain, i.e. in an antibody Fab (fragment antigen binding), the domain sequence deviates from the sequence in a native antibody in that at least one heavy chain domain is substituted by its corresponding light chain domain and vice versa. There are three general types of domain crossovers, (i) the crossover of the CH1 and the CL domains, which leads by the domain crossover in the light chain to a VL-CH1 domain sequence and by the domain crossover in the heavy chain fragment to a VH-CL domain sequence (or a full length antibody heavy chain with a VH-CL-hinge-CH2-CH3 domain sequence), (ii) the domain crossover of the VH and the VL domains, which leads by the domain crossover in the light chain to a VH-CL domain sequence and by the domain crossover in the heavy chain fragment to a VL-CH1 domain sequence, and (iii) the domain crossover of the complete light chain (VL-CL) and the complete VH-CH1 heavy chain fragment ("Fab crossover"), which leads to by domain crossover to a light chain with a VH-CH1 domain sequence and by domain crossover to a heavy chain fragment with a VL-CL domain sequence (all aforementioned domain sequences are indicated in N-terminal to C-terminal direction).

As used herein the term "replaced by each other" with respect to corresponding heavy and light chain domains refers to the aforementioned domain crossovers. As such, when CH1 and CL domains are "replaced by each other" it is referred to the domain crossover mentioned under item (i) and the resulting heavy and light chain domain sequence. Accordingly, when VH and VL are "replaced by each other" it is referred to the domain crossover mentioned under item (ii); and when the CH1 and CL domains are "replaced by each other" and the VH and VL domains are "replaced by each other" it is referred to the domain crossover mentioned under item (iii). Bispecific antibodies including domain crossovers are reported, e.g. in WO 2009/080251, WO 2009/080252, WO 2009/080253, WO 2009/080254 and Schaefer, W., et al, Proc. Natl. Acad. Sci USA 108 (2011) 11187-11192. Such antibodies are generally termed CrossMab.

Multispecific antibodies also comprise in one embodiment at least one Fab fragment including either a domain crossover of the CH1 and the CL domains as mentioned under item (i) above, or a domain crossover of the VH and the VL domains as mentioned under item (ii) above, or a domain crossover of the VH-CH1 and the VL-VL domains as mentioned under item (iii) above. In case of multispecific antibodies with domain crossover, the Fabs specifically binding to the same antigen(s) are constructed to be of the same domain sequence. Hence, in case more than one Fab with a domain crossover is contained in the multispecific antibody, said Fab(s) specifically bind to the same antigen.

A "humanized" antibody refers to an antibody comprising amino acid residues from non-human HVRs and amino acid residues from human FRs. In certain embodiments, a humanized antibody will comprise substantially all of at least one, and typically two, variable domains, in which all or substantially all of the HVRs (e.g., the CDRs) correspond to those of a non-human antibody, and all or substantially all of the FRs correspond to those of a human antibody. A humanized antibody optionally may comprise at least a portion of an antibody constant region derived from a human antibody. A "humanized form" of an antibody, e.g., a non-human antibody, refers to an antibody that has undergone humanization.

The term "recombinant antibody", as used herein, denotes all antibodies (chimeric, humanized and human) that are prepared, expressed, created or isolated by recombinant means, such as recombinant cells. This includes antibodies isolated from recombinant cells such as NS0, HEK, BHK or CHO cells.

As used herein, the term "antibody fragment" refers to a molecule other than an intact antibody that comprises a portion of an intact antibody that binds the antigen to which the intact antibody binds, i.e. it is a functional fragment. Examples of antibody fragments include but are not limited to Fv; Fab; Fab'; Fab'-SH; F(ab')2; bispecific Fab; diabodies; linear antibodies; single-chain antibody molecules (e.g., scFv or scFab).

As used herein, the term "antibody fragment" refers to a molecule other than an intact antibody that comprises a portion of an intact antibody that binds the antigen to which the intact antibody binds, i.e. it is a functional fragment. Examples of antibody fragments include but are not limited to Fv; Fab; Fab'; Fab'-SH; F(ab')2; bispecific Fab; diabodies; linear antibodies; single-chain antibody molecules (e.g., scFv or scFab).

A "target cell antigen" as used herein refers to an antigenic determinant presented on the surface of a target cell, for example a cell in a tumor such as a cancer cell or a cell of the tumor stroma. In certain embodiments, the target cell antigen is an antigen on the surface of a tumor cell. In one embodiment, target cell antigen is selected from the group consisting of Fibroblast Activation Protein (FAP), Carcinoembryonic Antigen (CEA), Melanoma-associated Chondroitin Sulfate Proteoglycan (MCSP), Epidermal Growth Factor Receptor (EGFR), CD19, CD20 and CD33. In particular, the target cell antigen is Fibroblast Activation Protein (FAP).

The term "CD19" refers to B-lymphocyte antigen CD19, also known as B-lymphocyte surface antigen B4 or T-cell surface antigen Leu-12 and includes any native CD19 from any vertebrate source, including mammals such as primates (e.g. humans) non-human primates (e.g. cynomolgus monkeys) and rodents (e.g. mice and rats), unless otherwise indicated. The amino acid sequence of human CD19 is shown in UniProt accession no. P15391 (version 160, SEQ ID NO: 103). The term encompasses "full-length" unprocessed human CD19 as well as any form of human CD19 that results from processing in the cell as long as the antibody as reported herein binds thereto. CD19 is a structurally distinct cell surface receptor expressed on the surface of human B cells, including, but not limited to, pre-B cells, B cells in early development {i.e., immature B cells), mature B cells through terminal differentiation into plasma cells, and malignant B cells. CD19 is expressed by most pre-B acute lymphoblastic leukemia (ALL), non-Hodgkin's lymphomas, B cell chronic lymphocytic leukemia (CLL), pro-lymphocytic leukemia, hairy cell leukemia, common acute lymphocytic leukemia, and some Null-acute lymphoblastic leukemia. The expression of CD19 on plasma cells further suggests it may be expressed on differentiated B cell tumors such as multiple myeloma. Therefore, the CD19 antigen is a target for immunotherapy in the treatment of non-Hodgkin's lymphoma, chronic lymphocytic leukemia and/or acute lymphoblastic leukemia.

The term "Fibroblast activation protein (FAP)", also known as Prolyl endopeptidase FAP or Seprase (EC 3.4.21), refers to any native FAP from any vertebrate source, including mammals such as primates (e.g. humans) non-human primates (e.g. cynomolgus monkeys) and rodents (e.g. mice and rats), unless otherwise indicated. The term encompasses "full-length", unprocessed FAP as well as any form of FAP which results from processing in the cell. The term also encompasses naturally occurring variants of FAP, e.g., splice variants or allelic variants. In one embodiment, the antigen binding molecule of the invention is capable of specific binding to human, mouse and/or cynomolgus FAP. The amino acid sequence of human FAP is shown in UniProt (www.uniprot.org) accession no. Q12884 (version 149, SEQ ID NO: 17), or NCBI (www.ncbi.nlm.nih.gov/) RefSeqNP_004451.2. The extracellular domain (ECD) of human FAP extends from amino acid position 26 to 760. The amino acid and nucleotide sequences of a His-tagged human FAP ECD is shown in SEQ ID NO: 14 and 15, respectively. The amino acid sequence of mouse FAP is shown in UniProt accession no. P97321 (version 126, SEQ ID NO: 18), or NCBI RefSeq NP_032012.1. The extracellular domain (ECD) of mouse FAP extends from amino acid position 26 to 761. SEQ ID NO: 19 and 20 show the amino acid and nucleotide sequences, respectively, of a His-tagged mouse FAP ECD. SEQ ID NO: 21 and 22 show the amino acid and nucleotide sequences, respectively, of a His-tagged cynomolgus FAP ECD. Preferably, an anti-FAP binding molecule of the invention binds to the extracellular domain of FAP. Exemplary anti-FAP binding molecules are described in WO 2012/020006.

The term "Carcinoembryonic antigen (CEA)", also known as Carcinoembryonic antigen-related cell adhesion molecule 5 (CEACAM5), refers to any native CEA from any vertebrate source, including mammals such as primates (e.g. humans) non-human primates (e.g. cynomolgus monkeys) and rodents (e.g. mice and rats), unless otherwise indicated. The amino acid sequence of human CEA is shown in UniProt accession no. P06731 (version 151, SEQ ID NO: 23). CEA has long been identified as a tumor-associated antigen (Gold and Freedman, J Exp. Med., 121:439-462, 1965; Berinstein N. L., J Clin. Oncol. 20:2197-2207, 2002). Originally classified as a protein expressed only in fetal tissue, CEA has now been identified in several normal adult tissues. These tissues are primarily epithelial in origin, including cells of the gastrointestinal, respiratory, and urogenital tracts, and cells of colon, cervix, sweat glands, and prostate (Nap et al., Tumour Biol., 9 (1988) 145-153; Nap et al., Cancer Res., 52 (1992) 2329-2339). Tumors of epithelial origin, as well as their metastases, contain CEA as a tumor associated antigen. While the presence of CEA itself does not indicate transformation to a cancerous cell, the distribution of CEA is indicative.

In normal tissue, CEA is generally expressed on the apical surface of the cell (Hammarström S., Semin. Cancer Biol. 9 (1999) 67-81), making it inaccessible to antibody in the blood stream. In contrast to normal tissue, CEA tends to be expressed over the entire surface of cancerous cells (Hammarström S., Semin Cancer Biol. 9 (1999) 67-81). This change of expression pattern makes CEA accessible to antibody binding in cancerous cells. In addition, CEA expression increases in cancerous cells. Furthermore, increased CEA expression promotes increased intercellular adhesions, which may lead to metastasis (Marshall J., Semin Oncol., 30 (2003) (a Suppl. 8) 30-36). The prevalence of CEA expression in various tumor entities is generally very high. In concordance with published data, own analyses performed in tissue samples confirmed its high prevalence, with approximately 95% in colorectal carcinoma (CRC), 90% in pancreatic cancer, 80% in gastric cancer, 60% in non-small cell lung cancer (NSCLC, where it is co-expressed with HER3), and 40% in breast cancer; low expression was found in small cell lung cancer and glioblastoma.

CEA is readily cleaved from the cell surface and shed into the blood stream from tumors, either directly or via the lymphatics. Because of this property, the level of serum CEA has been used as a clinical marker for diagnosis of cancers and screening for recurrence of cancers, particularly colorectal cancer (Goldenberg, D.M., Int. J. Biol. Mark. 7 (1992) 183-188; Chau I., et al., J. Clin. Oncol. 22 (2004) 420-1429; Flamini, et al., Clin. Cancer Res. 12 (2006) 6985-6988).

As used herein, the term "heterologous" indicates that a polypeptide does not originate from a specific cell and the respective encoding nucleic acid has been introduced into said cell by DNA delivery methods, e.g., by transfection, electroporation, or transformation methods. Thus, a heterologous polypeptide is a polypeptide that is artificial to the cell expressing it, whereby this is independent whether the polypeptide is a naturally occurring polypeptide originating from a different cell/organism or is a man-made polypeptide.

As used herein, the term "operably linked" refers to a juxtaposition of two or more components, wherein the components are in a relationship permitting them to function in their intended manner. For example, a promoter and/or an enhancer is operably linked to a coding sequence if the promoter and/or enhancer acts to modulate the transcription of the coding sequence. In certain embodiments, DNA sequences that are "operably linked" are contiguous and adjacent on a single chromosome. In certain embodiments, e.g., when it is necessary to join two protein encoding regions, such as a secretory leader and a polypeptide, the sequences are contiguous, adjacent, and in the same reading frame. In certain embodiments, an operably linked promoter is located upstream of the coding sequence and can be adjacent to it. In certain embodiments, e.g., with respect to enhancer sequences modulating the expression of a coding sequence, the two components can be operably linked although not adjacent. An enhancer is operably linked to a coding sequence if the enhancer increases transcription of the coding sequence. Operably linked enhancers can be located upstream, within, or downstream of coding sequences and can be located at a considerable distance from the promoter of the coding sequence. Operable linkage can be accomplished by recombinant methods known in the art, e.g., using PCR methodology and/or by ligation at convenient restriction sites. If convenient restriction sites do not exist, then synthetic oligonucleotide adaptors or linkers can be used in accord with conventional practice. An internal ribosomal entry site (IRES) is operably linked to an open reading frame (ORF) if it allows initiation of translation of the ORF at an internal location in a 5' end-independent manner.

### II. COMPOSITIONS AND METHODS

Generally, for the recombinant large-scale production of a polypeptide of interest, such as e.g. a therapeutic polypeptide, a cell stably expressing and secreting said polypeptide is required. This cell is termed "recombinant cell" or "recombinant production cell" and the process used for generating such a cell is termed "cell line development". In the first step of the cell line development process a suitable host cell, such as e.g. a CHO cell, is transfected with a nucleic acid sequence suitable for expression of said polypeptide of interest. In a second step, a cell stably expressing the polypeptide of interest is selected based on the co-expression of a selection marker, which had been co-transfected with the nucleic acid encoding the polypeptide of interest.

A nucleic acid encoding a polypeptide, i.e. the coding sequence, is called a structural gene. Such a structural gene is simple information and additional regulatory elements are required for expression thereof. Therefore, normally a structural gene is integrated in an expression cassette. The minimal regulatory elements needed for an expression cassette to be functional in a mammalian cell are a promoter functional in said mammalian cell, which is located upstream, i.e. 5', to the structural gene, and a polyadenylation signal sequence functional in said mammalian cell, which is located downstream, i.e. 3', to the structural gene. The promoter, the structural gene and the polyadenylation signal sequence are arranged in an operably linked form.

In case the polypeptide of interest is a heteromultimeric polypeptide that is composed of different (monomeric) polypeptides, not only a single expression cassette is required but a multitude of expression cassettes differing in the contained structural gene, i.e. at least one expression cassette for each of the different (monomeric) polypeptides of the heteromultimeric polypeptide. For example, an antibody-multimer-fusion polypeptide is a heteromultimeric polypeptide comprising one light chain, one heavy chain, one heavy chain constant domain-fusion polypeptide and one light chain constant domain fusion polypeptide. Thus, an antibody-multimer-fusion polypeptide is composed of four different polypeptides. Therefore, four expression cassettes are required for the expression of an antibody-multimer-fusion polypeptide, one for the light chain, one for the heavy chain, one for the heavy chain constant region fusion polypeptide and one for the light chain constant region fusion polypeptide.

The expression cassette(s) for the polypeptide of interest is(are) in turn integrated into a so called "expression vector". An "expression vector" is a nucleic acid providing all required elements for the amplification of said vector in bacterial cells as well as the expression of the comprised structural gene(s) in a mammalian cell. Typically, an expression vector comprises a prokaryotic plasmid propagation unit, e.g. for E. coli, comprising an origin of replication, and a prokaryotic selection marker, as well as a eukaryotic selection marker, and the expression cassettes required for the expression of the structural gene(s) of interest. An "expression vector" is a transport vehicle for the introduction of expression cassettes into a mammalian cell.

As outlined in the previous paragraphs, the more complex the polypeptide to be expressed is the higher also the number of required different expression cassettes is. Inherently with the number of expression cassettes, also the size of the nucleic acid to be integrated into the genome of the host cell increases. Concomitantly also the size of the expression vector increases. But there is a practical upper limit to the size of a vector in the range of about 15 kbps above which handling and processing efficiency profoundly drops. This issue can be addressed by using two or more expression vectors. Thereby the expression cassettes can be split between different expression vectors each comprising only some of the expression cassettes.

Generally, cell line development (CLD) relies on the random integration (RI) or targeted integration (TI) of the expression cassettes for the polypeptide of interest.

### II.a The method according to the current Invention

The current invention is based, at least in part, on the finding that for the expression of an antibody-multimer-fusion, which is a complex molecule comprising different polypeptides, i.e. which is a heteromultimer, the use of a defined expression cassette ratio results in efficient expression and production of the antibody-multimer-fusion in mammalian cells, such as CHO or HEK cells.

The current invention is based, at least in part, on the finding that for transient as well as stable expression of an antibody-multimer-fusion, which is a complex molecule comprising different polypeptides, i.e. which is a heteromultimer, the use of the same defined expression cassette ratio results in the highest expression yield and product quality.

The invention is based, at least in part, on the finding that for the expression of an antibody-multimer-fusion by a recombinant mammalian cell it is advantageous to use a ratio of the expression cassettes for the antibody heavy chain, the antibody light chain, the first fusion polypeptide and the second fusion polypeptide has to be a stoichiometric ratio of 1:1:2:1. By using this ratio, improved antibody-multimer-fusion expression with respect to yield and by-product formation can be obtained. It has further been found that this ratio is independent of the expression method, i.e. transient as well as stable cell lines are obtained with the same ratio, as well as the vector organization, i.e. using single expression cassette or multiple expression cassette vectors.

In the following the current invention is exemplified with an heteromultimeric antibody-multimer-fusion comprising a pair of an antibody heavy chain and an antibody light chain forming a binding site for human FAP and a first fusion polypeptide and a second fusion polypeptide wherein in the multimer is trimeric human 4-1-BBL. These experiments are presented solely for the illustration of the inventive concept and shall not be construed as limitation to the invention. Any pair of antibody chains and any multimeric polypeptide can likewise be used.

### Random integration, transient transfection, single expression cassette vectors

In a first set of experiments transient production of an anti-FAP antibody-4-1-BBL multimer-fusion was performed. A set of vectors, each comprising only a single expression cassette for a single polypeptide of the antibody-multimer-fusion, was used at different defined stoichiometric ratios. The results are presented in the following table. HC = antibody heavy chain, LC = antibody light chain, FH = first fusion polypeptide, FL = second fusion polypeptide, exp. = experiment number, eff. titer = effective titer (production of titer and main peak), rel. eff. titer = relative effective titer (relative titer normalized to an expression cassette ratio of 1:1:2:2).

| exp. | HC | LC | FH | FL | titer [µg/mL] | eff. titer | rel. eff titer |
|---|---|---|---|---|---|---|---|
| 1 | 1 | 1 | 1 | 1 | 16 | 14.38 | 107 % |
| 2 | 1 | 1 | 2 | 1 | 19 | 18.11 | 135 % |
| 3 | 1 | 1 | 2 | 2 | 14 | 13.38 | 100 % |
| 4 | 1 | 1 | 3 | 2 | 16 | 14.54 | 109 % |

It can be seen that an expression cassette ratio of 1:1:2:1 results in the best result and an overall 20 % higher effective titer as well as a 35 % higher effective titer than the 1:1:2:2 expression cassette ratio.

### Random integration, transient transfection, multiple expression cassette vectors

In a second set of experiments transient production of the anti-FAP antibody-4-1-BBL multimer-fusion was performed. A set of vectors, comprising one or two expression cassettes each for a single polypeptide of the antibody-multimer-fusion, was used at different defined stoichiometric ratios. The results are presented in the following table. HC = antibody heavy chain, LC = antibody light chain, FH = first fusion polypeptide, FL = second fusion polypeptide, exp. = experiment number, eff. titer = effective titer (production of titer and main peak), rel. eff. titer = relative effective titer (relative titer normalized to an expression cassette ratio of 1:1:2:2).

| exp. | vector 1 | vector 2 | vector 3 | HC | LC | FH | FL | titer [µg/ mL] | eff. titer | rel. eff titer |
|---|---|---|---|---|---|---|---|---|---|---|
| 1 | 1 | 1 | 1 | 1 | 1 | 2 | 1 | 17 | 14.6 | 167% |
| 2 | 1 | 2 | 1 | 1 | 1 | 3 | 2 | 12 | 10.2 | 117 % |
| 3 | 1 | 2 | - | 1 | 1 | 2 | 2 | 10 | 8.7 | 100 % |
| 4 | 1 | 3 | - | 1 | 1 | 3 | 3 | 9 | 7.8 | 89% |

| | | | | | | | | | | |
|---|---|---|---|---|---|---|---|---|---|---|
| vector 1 = double expression cassette vector with expression cassettes for HC and LC; vector 2 = double expression cassette vector with expression cassettes for FH and FL; vector 3 = single expression cassette vector with expression cassette for FH | | | | | | | | | | |

It can be seen that an expression cassette ratio of 1:1:2:1 results in in the best result and an overall 40 % higher effective titer as well as a 67 % higher effective titer than the 1:1:2:2 expression cassette ratio.

### Stable random integration, multiple expression cassette vectors

In a third set of experiments stable production of the anti-FAP antibody-4-1-BBL multimer-fusion was performed. A set of vectors, comprising one or two expression cassettes each for a single polypeptide of the antibody-multimer-fusion, was used at different defined stoichiometric ratios. The results are presented in the following table. HC = antibody heavy chain, LC = antibody light chain, FH = first fusion polypeptide, FL = second fusion polypeptide, exp. = experiment number, eff. titer = effective titer (production of titer and main peak), rel. eff. titer = relative effective titer (relative titer normalized to an expression cassette ratio of 1:1:2:2).

| exp. | vector 1 | vector 2 | vector 3 | HC | LC | FH | FL |
|---|---|---|---|---|---|---|---|
| 1 | 1 | 1 | 1 | 1 | 1 | 2 | 1 |
| 2 | 1 | 2 | - | 1 | 1 | 2 | 2 |

| | | | | | | | |
|---|---|---|---|---|---|---|---|
| vector 1 = double expression cassette vector with expression cassettes for HC and LC; vector 2 = double expression cassette vector with expression cassettes for FH and FL; vector 3 = single expression cassette vector with expression cassette for FH | | | | | | | |

Sixty plates with single cell clones were cultivated for each of the ratios of experiment 1 and experiment 2. Recovery rate, titer and product quality of the wells of the plates as well as batch cultivation product quality (%main-peak in CE-SDS) were analyzed. The results are presented in the following tables.

| plasmid ratio | titer positive wells | distribution confluence [categorical] | | | |
|---|---|---|---|---|---|
| | | 5-10% | 10-20% | 20-50% | ≥50% |
| 1:1+1 | 1748 | 4085 | 712 | 409 | 132 |
| 1:2 | 786 | 2658 | 298 | 170 | 57 |

| plasmid ratio | confluence positive wells (≥20%) | % titer positive wells of screened wells | distribution confluence [cumulated] in % of all wells | | | |
|---|---|---|---|---|---|---|
| | | | ≥5% | ≥10% | ≥20% | ≥50% |
| 1:1+1 | 541 | 11 | 33.7 | 7.9 | 3.4 | 0.8 |
| 1:2 | 227 | 3.8 | 15.3 | 2.5 | 1.1 | 0.3 |

It can be seen that for the 1:1:2:1 expression cassette ratio (1:1+1 vector ratio) a better growth (recovery), a double number of wells with more than 20% confluence as well as a double number of titer positive clones compared to the 1:1:2:2 expression cassette ratio (1:2 vector ratio) is obtained.

In more detail, the selection was based on confluence and titer (>5% confluence and IgG positive). All titer positive clones were processed to next selection steps. The double number of clones were derived from the plates comprising cell transfected with the 1:1:2:1 expression cassette ratio (1:1+1 vector ratio).

The total 1056 clones were selected for further ELISA retest analysis. Therein one third are clones obtained with the expression cassette ratio of 1:1:2:2 (vector ratio 1:2; 352 clones) and two third are clones obtained with the expression cassette ratio of 1:1:2:1 (vector ratio 1:1+1, 704 clones).

The second selection was based on ELISA binding and bridging assay.

Of the total 1056 clones 220 clones were found to express both parts of the antibody-multimer-fusion (main-product). Four times more clones were derived from the expression cassette ratio of 1:1:2:1 (vector ratio 1:1+1): 20% (45 clones) is from expression cassette ratio 1:1:2:2 (vector ratio 1:2) and 80% (175 clones) is from expression cassette ratio 1:1:2:1 (plasmid ratio 1:1:1 (1:1+1)).

In summary:
(45/352)^{∗}100%=12.78% of clones obtained with an expression cassette ratio of 1:1:2:2: (plasmid ratio 1:2) show have good product quality in ELISA re-test,
whereas
(175/704)^{∗}100%=24.86% of clones obtained with an expression cassette ratio of 1:1:2:1 (vector ratio of 1:1:1) show good product quality in ELISA re-test.

Thus, also in stable transfection the results obtained in transient transfection are confirmed.

### Random integration, transient transfection, single expression cassette vectors

In a fourth set of experiments transient production of an anti-CEA antibody-4-1-BBL multimer-fusion was performed. A set of vectors, each comprising a single expression cassette was used at different defined stoichiometric ratios. The results are presented in the following table. HC = antibody heavy chain, LC = antibody light chain, FH = first fusion polypeptide, FL = second fusion polypeptide, exp. = experiment number, eff. titer = effective titer (production of titer and main peak), rel. eff. titer = relative effective titer (relative titer normalized to an expression cassette ratio of 1:1:2:2).

| exp. | HC | LC | FH | FL | titer [µg/mL] | eff. titer | rel. eff titer |
|---|---|---|---|---|---|---|---|
| 1 | 1 | 1 | 1 | 1 | 144 | 108 | 107 |
| 2 | 1 | 1 | 2 | 1 | 148 | 124 | 124 |
| 3 | 1 | 1 | 2 | 2 | 117 | 101 | 100 |
| 4 | 2 | 1 | 2 | 2 | 62 | 53 | 52 |
| 5 | 1 | 1 | 3 | 2 | 121 | 103 | 102 |
| 6 | 2 | 1 | 3 | 1 | 92 | 80 | 80 |

It can be seen that an expression cassette ratio of 1:1:2:1 results in the best result and an overall 15 % higher effective titer as well as a 24 % higher effective titer than the 1:1:2:2 expression cassette ratio.

### Targeted integration, stable transfection, double RMCE

In a fifth set of experiments stable production of an anti-FAP antibody-4-1-BBL multimer-fusion was performed using targeted integration. A set of vectors, i.e. a front and a back vector, has been used. The targeted integration was performed using a double recombinase mediated cassette exchange reaction with Cre-recombinase. The front and the back vector comprised different expression cassettes as outlined in the following table with HC = antibody heavy chain, LC = antibody light chain, FH = first fusion polypeptide, FL = second fusion polypeptide.

| exp. | front vector | | | | back vector | | |
|---|---|---|---|---|---|---|---|
| 1 | FH | FL | FL | - | HC | LC | - |
| 2 | FH | FL | FH | FL | HC | LC | - |
| 3 | FH | FL | FH | FL | HC | LC | LC |
| 4 | FH | FH | FL | - | HC | LC | - |

Based on the different number of expression cassettes comprised in the front and back vector, respectively, different defined stoichiometric ratios were used. The results obtained from stable transfected cell pools are presented in the following table (n=2). HC = antibody heavy chain, LC = antibody light chain, FH = first fusion polypeptide, FL = second fusion polypeptide, exp. = experiment number, eff. titer = effective titer (production of titer and main peak), rel. eff. titer = relative effective titer (relative titer normalized to an expression cassette ratio of 1:1:2:2).

| exp. | HC | LC | FH | FL | titer [g/L] | eff. titer | rel. eff titer |
|---|---|---|---|---|---|---|---|
| 1 | 1 | 1 | 1 | 2 | 0.60 | 0.48 | 68 |
| 2 | 1 | 1 | 2 | 2 | 0.89 | 0.71 | 100 |
| 3 | 1 | 2 | 2 | 2 | 0.91 | 0.43 | 61 |
| 4 | 1 | 1 | 2 | 1 | 1.29 | 1.03 | 145 |

It can be seen that an expression cassette ratio of 1:1:2:1 results in the best result and an overall 45 % higher effective titer as well as a 45 % higher effective titer then the 1:1:2:2 expression cassette ratio.

### Random integration, transient transfection, single expression cassette vectors

In a sixth set of experiments transient production of an anti-CD19 antibody-4-1-BBL multimer-fusion was performed. A set of vectors, each comprising only a single expression cassette for a single polypeptide of the antibody-multimer-fusion, was used at different defined stoichiometric ratios. The results are presented in the following table. HC = antibody heavy chain, LC = antibody light chain, FH = first fusion polypeptide, FL = second fusion polypeptide, exp. = experiment number, eff. titer = effective titer (production of titer and main peak), rel. eff. titer = relative effective titer (relative titer normalized to an expression cassette ratio of 1:1:2:2).

| exp. | HC | LC | FH | FL | titer [µg/mL] | eff. titer | rel. eff titer |
|---|---|---|---|---|---|---|---|
| 1 | 1 | 1 | 1 | 1 | 14 | 12.04 | 114 % |
| 2 | 1 | 1 | 2 | 1 | 16 | 12.96 | 123 % |
| 3 | 1 | 1 | 2 | 2 | 16 | 10.56 | 100 % |
| 4 | 1 | 1 | 3 | 2 | 12 | 9.6 | 91 % |

It can be seen that an expression cassette ratio of 1:1:2:1 results in the best result and an overall 9 % higher effective titer as well as a 23 % higher effective titer than the 1:1:2:2 expression cassette ratio.

### Summary

Thus, independent from the method used for the generation of the cell line, an expression cassette ratio of 1:1:2:1 (HC:LC:FH:FL) results in improved product quality, i.e. effective titer. Additionally, an increase in the number of suitable stable clones for expressing an antibody-multimer-fusion can be obtained.

### II.b Ligands interacting with molecules of the TNF

Ligands interacting with molecules of the TNF (tumor necrosis factor) receptor superfamily have pivotal roles in the organization and function of the immune system. While regulating normal functions such as immune responses, hematopoiesis and morphogenesis, the TNF family ligands (also called cytokines) play a role in tumorigenesis, transplant rejection, septic shock, viral replication, bone resorption, rheumatoid arthritis and diabetes (Aggarwal, 2003). The TNF ligand family comprises 18 genes encoding 19 type II (i.e. intracellular N terminus and extracellular C-terminus) transmembrane proteins, characterized by the presence of a conserved C-terminal domain coined the 'TNF homology domain' (THD). This domain is responsible for receptor binding and is thus critical for the biological activity of the TNF ligand family members. The sequence identity between family members is ~20―30% (Bodmer, 2002). Members of the TNF ligand family exert their biological function as self-assembling, noncovalent trimers (Banner et al, Cell 73 (1993) 431-445). Thus, the TNF family ligands form a trimer that is able to bind to and to activate the corresponding receptors of TNFR superfamily.

4-1-BB (CD137), a member of the TNF receptor superfamily, has been first identified as a molecule whose expression is induced by T-cell activation (Kwon and Weissman, 1989). Subsequent studies demonstrated expression of 4-1-BB in T- and B-lymphocytes (Snell et al., 2011; Zhang et al., 2010), NK-cells (Lin et al., 2008), NKT-cells (Kim et al., 2008), monocytes (Kienzle and von Kempis, 2000; Schwarz et al., 1995), neutrophils (Heinisch et al., 2000), mast (Nishimoto et al., 2005) and dendritic cells as well as cells of non-hematopoietic origin such as endothelial and smooth muscle cells (Broll et al., 2001; Olofsson et al., 2008). Expression of 4-1-BB in different cell types is mostly inducible and driven by various stimulatory signals, such as T-cell receptor (TCR) or B-cell receptor triggering, as well as signaling induced through co-stimulatory molecules or receptors of pro-inflammatory cytokines (Diehl et al., 2002; von Kempis et al., 1997; Zhang et al., 2010).

Expression of 4-1-BB ligand (4-1-BBL or CD137L) is more restricted and is observed on professional antigen presenting cells (APC) such as B-cells, dendritic cells (DCs) and macrophages. Inducible expression of 4-1-BBL is characteristic for T-cells, including both αβ and γδ T-cell subsets, and endothelial cells (reviewed in Shao and Schwarz, 2011).

CD137 signaling is known to stimulate IFNγ secretion and proliferation of NK cells (Buechele et al., 2012; Lin et al., 2008; Melero et al., 1998) as well as to promote DC activation as indicated by their increased survival and capacity to secret cytokines and upregulate co-stimulatory molecules (Choi et al., 2009; Futagawa et al., 2002; Wilcox et al., 2002). However, CD137 is best characterized as a co-stimulatory molecule, which modulates TCR-induced activation in both the CD4+ and CD8+ subsets of T-cells. In combination with TCR triggering, agonistic 4-1-BB-specific antibodies enhance proliferation of T-cells, stimulate lymphokine secretion and decrease sensitivity of T-lymphocytes to activation-induced cells death (reviewed in Snell et al., 2011).

In line with these co-stimulatory effects of 4-1-BB antibodies on T-cells in vitro, their administration to tumor bearing mice leads to potent anti-tumor effects in many experimental tumor models (Melero et al., 1997; Narazaki et al., 2010). However, 4-1-BB usually exhibits its potency as an anti-tumor agent only when administered in combination with other immunomodulatory compounds (Curran et al., 2011; Guo et al., 2013; Morales-Kastresana et al., 2013; Teng et al., 2009; Wei et al., 2013), chemotherapeutic reagents (Ju et al., 2008; Kim et al., 2009), tumor-specific vaccination (Cuadros et al., 2005; Lee et al., 2011) or radiotherapy (Shi and Siemann, 2006). In vivo depletion experiments demonstrated that CD8+ T-cells play the most critical role in anti-tumoral effect of 4-1-BB-specific antibodies. However, depending on the tumor model or combination therapy, which includes anti-4-1-BB, contributions of other types of cells such as DCs, NK-cells or CD4+ T-cells have been reported (Melero et al., 1997; Murillo et al., 2009; Narazaki et al., 2010; Stagg et al., 2011).

In addition to their direct effects on different lymphocyte subsets, 4-1-BB agonists can also induce infiltration and retention of activated T-cells in the tumor through 4-1-BB-mediated upregulation of intercellular adhesion molecule 1 (ICAM1) and vascular cell adhesion molecule 1 (VCAM1) on tumor vascular endothelium (Palazon et al., 2011).

4-1-BB triggering may also reverse the state of T-cell anergy induced by exposure to soluble antigen that may contribute to disruption of immunological tolerance in the tumor micro-environment or during chronic infections (Wilcox et al., 2004).

It appears that the immunomodulatory properties of 4-1-BB agonistic antibodies in vivo require the presence of the wild type Fc-portion on the antibody molecule thereby implicating Fc-receptor binding as an important event required for the pharmacological activity of such reagents as has been described for agonistic antibodies specific to other apoptosis-inducing or immunomodulatory members of the TNFR-superfamily (Li and Ravetch, 2011; Teng et al., 2009). However, systemic administration of 4-1-BB-specific agonistic antibodies with the functionally active Fc domain also induces expansion of CD8+ T-cells associated with liver toxicity (Dubrot et al., 2010) that is diminished or significantly ameliorated in the absence of functional Fc-receptors in mice. In human clinical trials (ClinicalTrials.gov, NCT00309023), Fc-competent 4-1-BB agonistic antibodies (BMS-663513) administered once every three weeks for 12 weeks induced stabilization of the disease in patients with melanoma, ovarian or renal cell carcinoma. However, the same antibody given in another trial (NCT00612664) caused grade 4 hepatitis leading to termination of the trial (Simeone and Ascierto, 2012).

Collectively, the available pre-clinical and clinical data clearly demonstrate that there is a high clinical need for effective 4-1-BB agonists. However, new generation drug candidates should not only effectively engage 4-1-BB on the surface of hematopoietic and endothelial cells but also be capable of achieving that through mechanisms other than binding to Fc-receptors in order to avoid uncontrollable side effects. The latter may be accomplished through preferential binding to and oligomerization on tumor-specific or tumor-associated moieties.

Fusion proteins composed of one extracellular domain of a 4-1-BB ligand and a single chain antibody fragment (Mueller et al., 2008; Hornig et al., 2012) or a single 4-1-BB ligand fused to the C-terminus of a heavy chain (Zhang et al, 2007) have been made. WO 2010/010051 discloses the generation of fusion proteins that consist of three TNF ligand ectodomains linked to each other and fused to an antibody part.

However, there is still a need of new antigen binding molecules that combine a moiety capable of preferred binding to tumor-specific or tumor-associated targets with a moiety capable of forming a costimulatory TNF ligand trimer and that have sufficient stability to be pharmaceutically useful. The antigen binding molecules of the present invention comprise both and surprisingly they provide a trimeric and thus biologically active TNF ligand, although one of the trimerising TNF ligand ectodomains is located on another polypeptide than the other two TNF ligand ectodomains of the molecule.

### II.c Recombinant Methods and Compositions

Antibodies may be produced using recombinant methods and compositions, e.g., as described in US 4,816,567. For these methods, one or more isolated nucleic acid(s) encoding an antibody are provided.

In one aspect, a method of making an antibody-multimer-fusion polypeptide is provided, wherein the method comprises culturing a host cell comprising nucleic acid(s) encoding the antibody-multimer-fusion polypeptide obtained with a method according to the invention under conditions suitable for expression of the antibody-multimer-fusion polypeptide, and optionally recovering the antibody-multimer-fusion polypeptide from the host cell (or host cell culture medium).

For recombinant production of an antibody-multimer-fusion polypeptide, nucleic acids encoding the antibody-multimer-fusion polypeptide, e.g., as described herein, are isolated and inserted into one or more vectors for further cloning and/or expression in a host cell. Such nucleic acids may be readily isolated and sequenced using conventional procedures or produced by recombinant methods or obtained by chemical synthesis.

Generally, for the recombinant large-scale production of a polypeptide of interest, such as e.g. a therapeutic antibody-multimer-fusion polypeptide, a cell stably expressing and secreting said polypeptide is required. This cell is termed "recombinant cell" or "recombinant production cell" and the process used for generating such a cell is termed "cell line development". In the first step of the cell line development process, a suitable host cell, such as e.g. a CHO cell, is transfected with a nucleic acid sequence suitable for expression of said polypeptide of interest. In a second step, a cell stably expressing the polypeptide of interest is selected based on the co-expression of a selection marker, which had been co-transfected with the nucleic acid encoding the polypeptide of interest.

A nucleic acid encoding a polypeptide, i.e. the coding sequence, is called a structural gene. Such a structural gene is simple information and additional regulatory elements are required for expression thereof. Therefore, normally a structural gene is integrated in a so-called expression cassette. The minimal regulatory elements needed for an expression cassette to be functional in a mammalian cell are a promoter functional in said mammalian cell, which is located upstream, i.e. 5', to the structural gene, and a polyadenylation signal sequence functional in said mammalian cell, which is located downstream, i.e. 3', to the structural gene. The promoter, the structural gene and the polyadenylation signal sequence are arranged in an operably linked form.

As outlined in the previous paragraphs, the more complex the polypeptide to be expressed is the higher also the number of required different expression cassettes gets. Inherently with the number of expression cassettes also the size of the nucleic acid to be integrated into the genome of the host cell increases. Concomitantly also the size of the expression vector increases. However, there is a practical upper limit to the size of a vector in the range of about 15 kbps above which handling and processing efficiency profoundly drops. This issue can be addressed by using two or more expression vectors. Thereby the expression cassettes can be split between different expression vectors each comprising only some of the expression cassettes resulting in a size reduction.

Cell line development (CLD) for the generation of recombinant cell expressing a heterologous polypeptide, such as e.g. an antibody-multimer-fusion polypeptide, employs either random integration (RI) or targeted integration (TI) of the nucleic acid(s) comprising the respective expression cassettes required for the expression and production of the heterologous antibody-multimer-fusion polypeptide of interest.

Using RI, in general, several vectors or fragments thereof integrate into the cell's genome at the same or different loci.

Using TI, in general, a single copy of the transgene comprising the different expression cassettes is integrated at a predetermined "hot-spot" in the host cell's genome.

Suitable host cells for the expression of an (glycosylated) antibody are generally derived from multicellular organisms such as e.g. vertebrates.

### II.d Host cells

Any mammalian cell line that is adapted to grow in suspension can be used in the method according to the current invention. In addition, independent from the integration method, i.e. for RI as well as TI, any mammalian host cell can be used.

Examples of useful mammalian host cell lines are human amniocyte cells (e.g. CAP-T cells as described in Woelfel, J. et al., BMC Proc. 5 (2011) P133); monkey kidney CV1 line transformed by SV40 (COS-7); human embryonic kidney line (HEK293 or HEK293T cells as described, e.g., in Graham, F.L. et al., J. Gen Virol. 36 (1977) 59-74); baby hamster kidney cells (BHK); mouse sertoli cells (TM4 cells as described, e.g., in Mather, J.P., Biol. Reprod. 23 (1980) 243-252); monkey kidney cells (CV1); African green monkey kidney cells (VERO-76); human cervical carcinoma cells (HELA); canine kidney cells (MDCK; buffalo rat liver cells (BRL 3A); human lung cells (W138); human liver cells (Hep G2); mouse mammary tumor (MMT 060562); TRI cells (as described, e.g., in Mather, J.P. et al., Annals N.Y. Acad. Sci. 383 (1982) 44-68); MRC 5 cells; and FS4 cells. Other useful mammalian host cell lines include Chinese hamster ovary (CHO) cells, including DHFR-CHO cells (Urlaub, G. et al., Proc. Natl. Acad. Sci. USA 77 (1980) 4216-4220); and myeloma cell lines such as Y0, NS0 and Sp2/0. For a review of certain mammalian host cell lines suitable for antibody production, see, e.g., Yazaki, P. and Wu, A.M., Methods in Molecular Biology, Vol. 248, Lo, B.K.C. (ed.), Humana Press, Totowa, NJ (2004), pp. 255-268.

In one embodiment, the mammalian host cell is, e.g., a Chinese Hamster Ovary (CHO) cell (e.g. CHO K1, CHO DG44, etc.), a Human Embryonic Kidney (HEK) cell, a lymphoid cell (e.g., Y0, NS0, Sp20 cell), or a human amniocyte cells (e.g. CAP-T, etc.). In one preferred embodiment, the mammalian host cell is a CHO cell.

Targeted integration allows exogenous nucleotide sequences to be integrated into a pre-determined site of a mammalian cell's genome. In certain embodiments, the targeted integration is mediated by a recombinase that recognizes one or more recombination recognition sequences (RRSs), which are present in the genome and in the exogenous nucleotide sequence to be integrated. In certain embodiments, the targeted integration is mediated by homologous recombination.

A "recombination recognition sequence" (RRS) is a nucleotide sequence recognized by a recombinase and is necessary and sufficient for recombinase-mediated recombination events. A RRS can be used to define the position where a recombination event will occur in a nucleotide sequence.

In certain embodiments, a RRS can be recognized by a Cre recombinase. In certain embodiments, a RRS can be recognized by a FLP recombinase. In certain embodiments, a RRS can be recognized by a Bxb1 integrase. In certain embodiments, a RRS can be recognized by a ϕC31 integrase.

In certain embodiments when the RRS is a LoxP site, the cell requires the Cre recombinase to perform the recombination. In certain embodiments when the RRS is a FRT site, the cell requires the FLP recombinase to perform the recombination. In certain embodiments when the RRS is a Bxb1 attP or a Bxb1 attB site, the cell requires the Bxb1 integrase to perform the recombination. In certain embodiments when the RRS is a ϕC31 attP or a ϕC31 attB site, the cell requires the ϕC31 integrase to perform the recombination. The recombinases can be introduced into a cell using an expression vector comprising coding sequences of the enzymes or as protein or an mRNA.

With respect to TI, any known or future mammalian host cell suitable for TI comprising a landing site as described herein integrated at a single site within a locus of the genome can be used in the current invention. Such a cell is denoted as mammalian TI host cell. In certain embodiments, the mammalian TI host cell is a hamster cell, a human cell, a rat cell, or a mouse cell comprising a landing site as described herein. In one preferred embodiment, the mammalian TI host cell is a CHO cell. In certain embodiments, the mammalian TI host cell is a Chinese hamster ovary (CHO) cell, a CHO K1 cell, a CHO K1SV cell, a CHO DG44 cell, a CHO DUKXB-11 cell, a CHO K1S cell, or a CHO K1 M cell comprising a landing site as described herein integrated at a single site within a locus of the genome.

In certain embodiments, a mammalian TI host cell comprises an integrated landing site, wherein the landing site comprises one or more recombination recognition sequence (RRS). The RRS can be recognized by a recombinase, for example, a Cre recombinase, an FLP recombinase, a Bxb1 integrase, or a ϕC31 integrase. The RRS can be selected independently of each other from the group consisting of a LoxP sequence, a LoxP L3 sequence, a LoxP 2L sequence, a LoxFas sequence, a Lox511 sequence, a Lox2272 sequence, a Lox2372 sequence, a Lox5171 sequence, a Loxm2 sequence, a Lox71 sequence, a Lox66 sequence, a FRT sequence, a Bxb1 attP sequence, a Bxb1 attB sequence, a ϕC31 attP sequence, and a ϕC31 attB sequence. If multiple RRSs have to be present, the selection of each of the sequences is dependent on the other insofar as non-identical RRSs are chosen.

In certain embodiments, the landing site comprises one or more recombination recognition sequence (RRS), wherein the RRS can be recognized by a recombinase. In certain embodiments, the integrated landing site comprises at least two RRSs. In certain embodiments, an integrated landing site comprises three RRSs, wherein the third RRS is located between the first and the second RRS. In certain preferred embodiments, all three RRSs are different. In certain embodiments, the landing site comprises a first, a second and a third RRS, and at least one selection marker located between the first and the second RRS, and the third RRS is different from the first and/or the second RRS. In certain embodiments, the landing site further comprises a second selection marker, and the first and the second selection markers are different.

In certain embodiments, the landing site further comprises a third selection marker and an internal ribosome entry site (IRES), wherein the IRES is operably linked to the third selection marker. The third selection marker can be different from the first or the second selection marker.

Although the invention is exemplified with HEK and CHO cells herein, this is presented solely to exemplify the invention but shall not be construed in any way as limitation. The true scope of the invention is set forth in the claims.

An exemplary mammalian TI host cell that is suitable for use in a method according to the current invention is a CHO cell harboring a landing site integrated at a single site within a locus of its genome wherein the landing site comprises three heterospecific loxP sites for Cre recombinase mediated DNA recombination.

In this example, the heterospecific loxP sites are L3, LoxFas and 2L (see e.g. Lanza et al., Biotechnol. J. 7 (2012) 898-908; Wong et al., Nucleic Acids Res. 33 (2005) e147), whereby L3 and 2L flank the landing site at the 5'-end and 3'-end, respectively, and LoxFas is located between the L3 and 2L sites. The landing site further contains a bicistronic unit linking the expression of a selection marker via an IRES to the expression of the fluorescent GFP protein allowing to stabilize the landing site by positive selection as well as to select for the absence of the site after transfection and Cre-recombination (negative selection). Green fluorescence protein (GFP) serves for monitoring the RMCE reaction.

Such a configuration of the landing site as outlined in the previous paragraph allows for the simultaneous integration of two vectors, e.g. of a so called front vector harboring an L3 and a LoxFas site and a back vector harboring a LoxFas and an 2L site. The functional elements of a selection marker gene different from that present in the landing site can be distributed between both vectors: promoter and start codon can be located on the front vector whereas coding region and poly A signal are located on the back vector. Only correct recombinase-mediated integration of said nucleic acids from both vectors induces resistance against the respective selection agent.

Generally, a mammalian TI host cell is a mammalian cell comprising a landing site integrated at a single site within a locus of the genome of the mammalian cell, wherein the landing site comprises a first and a second recombination recognition sequence flanking at least one first selection marker, and a third recombination recognition sequence located between the first and the second recombination recognition sequence, and all the recombination recognition sequences are different.

The selection marker(s) can be selected from the group consisting of an aminoglycoside phosphotransferase (APH) (e.g., hygromycin phosphotransferase (HYG), neomycin and G418 APH), dihydrofolate reductase (DHFR), thymidine kinase (TK), glutamine synthetase (GS), asparagine synthetase, tryptophan synthetase (indole), histidinol dehydrogenase (histidinol D), and genes encoding resistance to puromycin, blasticidin, bleomycin, phleomycin, chloramphenicol, Zeocin, and mycophenolic acid. The selection marker(s) can also be a fluorescent protein selected from the group consisting of green fluorescent protein (GFP), enhanced GFP (eGFP), a synthetic GFP, yellow fluorescent protein (YFP), enhanced YFP (eYFP), cyan fluorescent protein (CFP), mPlum, mCherry, tdTomato, mStrawberry, J-red, DsRed-monomer, mOrange, mKO, mCitrine, Venus, YPet, Emerald6, CyPet, mCFPm, Cerulean, and T-Sapphire.

An exogenous nucleotide sequence is a nucleotide sequence that does not originate from a specific cell but can be introduced into said cell by DNA delivery methods, such as, e.g., by transfection, electroporation, or transformation methods. In certain embodiments, a mammalian TI host cell comprises at least one landing site integrated at one or more integration sites in the mammalian cell's genome. In certain embodiments, the landing site is integrated at one or more integration sites within a specific a locus of the genome of the mammalian cell.

In certain embodiments, the integrated landing site comprises at least one selection marker. In certain embodiments, the integrated landing site comprises a first, a second and a third RRS, and at least one selection marker. In certain embodiments, a selection marker is located between the first and the second RRS. In certain embodiments, two RRSs flank at least one selection marker, i.e., a first RRS is located 5' (upstream) and a second RRS is located 3' (downstream) of the selection marker. In certain embodiments, a first RRS is adjacent to the 5'-end of the selection marker and a second RRS is adjacent to the 3'-end of the selection marker. In certain embodiments, the landing site comprises a first, second, and third RRS, and at least one selection marker located between the first and the third RRS.

In certain embodiments, a selection marker is located between a first and a second RRS and the two flanking RRSs are different. In certain preferred embodiments, the first flanking RRS is a LoxP L3 sequence and the second flanking RRS is a LoxP 2L sequence. In certain embodiments, a LoxP L3 sequenced is located 5' of the selection marker and a LoxP 2L sequence is located 3' of the selection marker. In certain embodiments, the first flanking RRS is a wild-type FRT sequence and the second flanking RRS is a mutant FRT sequence. In certain embodiments, the first flanking RRS is a Bxb1 attP sequence and the second flanking RRS is a Bxb1 attB sequence. In certain embodiments, the first flanking RRS is a ϕC31 attP sequence and the second flanking RRS is a ϕC31 attB sequence. In certain embodiments, the two RRSs are positioned in the same orientation. In certain embodiments, the two RRSs are both in the forward or reverse orientation. In certain embodiments, the two RRSs are positioned in opposite orientation.

In certain embodiments, the integrated landing site comprises a first and a second selection marker, which are flanked by two RRSs, wherein the first selection marker is different from the second selection marker. In certain embodiments, the two selection markers are both independently of each other selected from the group consisting of a glutamine synthetase selection marker, a thymidine kinase selection marker, a HYG selection marker, and a puromycin resistance selection marker. In certain embodiments, the integrated landing site comprises a thymidine kinase selection marker and a HYG selection marker. In certain embodiments, the first selection maker is selected from the group consisting of an aminoglycoside phosphotransferase (APH) (e.g., hygromycin phosphotransferase (HYG), neomycin and G418 APH), dihydrofolate reductase (DHFR), thymidine kinase (TK), glutamine synthetase (GS), asparagine synthetase, tryptophan synthetase (indole), histidinol dehydrogenase (histidinol D), and genes encoding resistance to puromycin, blasticidin, bleomycin, phleomycin, chloramphenicol, Zeocin, and mycophenolic acid, and the second selection maker is selected from the group consisting of a GFP, an eGFP, a synthetic GFP, a YFP, an eYFP, a CFP, an mPlum, an mCherry, a tdTomato, an mStrawberry, a J-red, a DsRed-monomer, an mOrange, an mKO, an mCitrine, a Venus, a YPet, an Emerald, a CyPet, an mCFPm, a Cerulean, and a T-Sapphire fluorescent protein. In certain embodiments, the first selection marker is a glutamine synthetase selection marker and the second selection marker is a GFP fluorescent protein. In certain embodiments, the two RRSs flanking both selection markers are different.

In certain embodiments, the selection marker is operably linked to a promoter sequence. In certain embodiments, the selection marker is operably linked to an SV40 promoter. In certain embodiments, the selection marker is operably linked to a human Cytomegalovirus (CMV) promoter.

### II.e Targeted Integration

One method for the generation of a recombinant mammalian cell according to the current invention is targeted integration (TI).

In targeted integration, site-specific recombination is employed for the introduction of an exogenous nucleic acid into a specific locus in the genome of a mammalian TI host cell. This is an enzymatic process wherein a sequence at the site of integration in the genome is exchanged for the exogenous nucleic acid. One system used to effect such nucleic acid exchanges is the Cre-lox system. The enzyme catalyzing the exchange is the Cre recombinase. The sequence to be exchanged is defined by the position of two lox(P)-sites in the genome as well as in the exogenous nucleic acid. These lox(P)-sites are recognized by the Cre recombinase. Nothing more is required, i.e. no ATP etc. Originally, the Cre-lox system has been found in bacteriophage P1.

The Cre-lox system operates in different cell types, like mammals, plants, bacteria and yeast.

In one embodiment, the exogenous nucleic acid encoding the antibody-multimer-fusion polypeptide has been integrated into the mammalian TI host cell by single or double recombinase mediated cassette exchange (RMCE). Thereby a recombinant mammalian cell, such as a recombinant CHO cell, is obtained, in which a defined and specific expression cassette sequence has been integrated into the genome at a single locus, which in turn results in the efficient expression and production of the antibody-multimer-fusion polypeptide.

The Cre-LoxP site-specific recombination system has been widely used in many biological experimental systems. Cre recombinase is a 38-kDa site-specific DNA recombinase that recognizes 34 bp LoxP sequences. Cre recombinase is derived from bacteriophage P1 and belongs to the tyrosine family site-specific recombinase. Cre recombinase can mediate both intra and intermolecular recombination between LoxP sequences. The LoxP sequence is composed of an 8 bp non-palindromic core region flanked by two 13 bp inverted repeats. Cre recombinase binds to the 13 bp repeat thereby mediating recombination within the 8 bp core region. Cre-LoxP-mediated recombination occurs at a high efficiency and does not require any other host factors. If two LoxP sequences are placed in the same orientation on the same nucleotide sequence, Cre recombinase-mediated recombination will excise DNA sequences located between the two LoxP sequences as a covalently closed circle. If two LoxP sequences are placed in an inverted position on the same nucleotide sequence, Cre recombinase-mediated recombination will invert the orientation of the DNA sequences located between the two sequences. If two LoxP sequences are on two different DNA molecules and if one DNA molecule is circular, Cre recombinase-mediated recombination will result in integration of the circular DNA sequence.

The term "matching RRSs" indicates that a recombination occurs between two RRSs. In certain embodiments, the two matching RRSs are the same. In certain embodiments, both RRSs are wild-type LoxP sequences. In certain embodiments, both RRSs are mutant LoxP sequences. In certain embodiments, both RRSs are wild-type FRT sequences. In certain embodiments, both RRSs are mutant FRT sequences. In certain embodiments, the two matching RRSs are different sequences but can be recognized by the same recombinase. In certain embodiments, the first matching RRS is a Bxb1 attP sequence and the second matching RRS is a Bxb1 attB sequence. In certain embodiments, the first matching RRS is a ϕC31 attB sequence and the second matching RRS is a ϕC31 attB sequence.

In certain embodiments of the invention, a "two-plasmid RMCE" strategy or "double RMCE" is employed using a two-vector combination. For example, but not by way of limitation, an integrated landing site could comprise three RRSs, e.g., an arrangement where the third RRS ("RRS3") is present between the first RRS ("RRS1") and the second RRS ("RRS2"), while a first vector comprises two RRSs matching the first and the third RRS on the integrated exogenous nucleotide sequence, and a second vector comprises two RRSs matching the third and the second RRS on the integrated exogenous nucleotide sequence.

The two-plasmid RMCE strategy involves using three RRS sites to carry out two independent RMCEs simultaneously. Therefore, a landing site in the mammalian TI host cell using the two-plasmid RMCE strategy includes a third RRS site (RRS3) that has no cross activity with either the first RRS site (RRS1) or the second RRS site (RRS2). The two plasmids to be targeted require the same flanking RRS sites for efficient targeting, one plasmid (front) flanked by RRS1 and RRS3 and the other (back) by RRS3 and RRS2. In addition, two selection markers are needed in the two-plasmid RMCE. One selection marker expression cassette was split into two parts. The front plasmid would contain the promoter followed by a start codon and the RRS3 sequence. The back plasmid would have the RRS3 sequence fused to the N-terminus of the selection marker coding region, minus the start-codon (ATG). Additional nucleotides may need to be inserted between the RRS3 site and the selection marker sequence to ensure in frame translation for the fusion protein, i.e. operable linkage. Only when both plasmids are correctly inserted, the full expression cassette of the selection marker will be assembled and, thus, rendering cells resistance to the respective selection agent.

Two-plasmid RMCE involves double recombination cross-over events, catalyzed by a recombinase, between the two heterospecific RRSs within the target genomic locus and the donor DNA molecule. Two-plasmid RMCE is designed to introduce a copy of the DNA sequences from the front- and back-vector in combination into the predetermined locus of a mammalian TI host cell's genome. RMCE can be implemented such that prokaryotic vector sequences are not introduced into the mammalian TI host cell's genome, thus, reducing and/or preventing unwanted triggering of host immune or defense mechanisms. The RMCE procedure can be repeated with multiple DNA sequences.

In certain embodiments, targeted integration is achieved by two RMCEs, wherein two different DNA sequences, each comprising at least one expression cassette encoding a part of the antibody-multimer-fusion polypeptide and/or at least one selection marker or part thereof flanked by two heterospecific RRSs, are both integrated into a pre-determined site of the genome of a RRSs matching mammalian TI host cell. In certain embodiments, targeted integration is achieved by multiple RMCEs, wherein DNA sequences from multiple vectors, each comprising at least one expression cassette encoding a part of an antibody-multimer-fusion polypeptide and/or at least one selection marker or part thereof flanked by two heterospecific RRSs, are all integrated into a predetermined site of the genome of a mammalian TI host cell. In certain embodiments the selection marker can be partially encoded on the first the vector and partially encoded on the second vector such that only the correct integration of both by double RMCE allows for the expression of the selection marker.

In certain embodiments, targeted integration via recombinase-mediated recombination leads to selection marker and/or the different expression cassettes for the antibody-multimer-fusion polypeptide integrated into one or more predetermined integration sites of a host cell genome free of sequences from a prokaryotic vector.
- SEQ ID NO: 130:: exemplary sequence of an L3 recombinase recognition sequence
- SEQ ID NO: 131:: exemplary sequence of a 2L recombinase recognition sequence
- SEQ ID NO: 132:: exemplary sequence of a LoxFas recombinase recognition sequence
- SEQ ID NO: 133-5:: exemplary variants of human CMV promoter
- SEQ ID NO: 136:: exemplary SV40 polyadenylation signal sequence
- SEQ ID NO: 137:: exemplary bGH polyadenylation signal sequence
- SEQ ID NO: 138:: exemplary hGT terminator sequence
- SEQ ID NO: 139:: exemplary SV40 promoter sequence
- SEQ ID NO: 140:: exemplary GFP nucleic acid sequence

In addition to the various embodiments depicted and embodimented, the disclosed subject matter is also directed to other embodiments having other combinations of the features disclosed and embodimented herein. As such, the particular features presented herein can be combined with each other in other manners within the scope of the disclosed subject matter such that the disclosed subject matter includes any suitable combination of the features disclosed herein. The foregoing description of specific embodiments of the disclosed subject matter has been presented for purposes of illustration and description. It is not intended to be exhaustive or to limit the disclosed subject matter to those embodiments disclosed.

It will be apparent to those skilled in the art that various modifications and variations can be made in the compositions and methods of the disclosed subject matter without departing from the spirit or scope of the disclosed subject matter. Thus, it is intended that the disclosed subject matter include modifications and variations that are within the scope of the appended embodiments and their equivalents.

Various publications, patents and patent applications are cited herein, the contents of which are hereby incorporated by reference in their entireties.

The following examples and sequences are provided to aid the understanding of the present invention, the true scope of which is set forth in the appended embodiments.

### Citations

Ascierto, P. A., et al. Semin Oncol 37:508-516.
Aggarwal B.B., Nat. Rev. Immunol. 3 (2003) 745-56.
Banner D. et al., Cell 73 (1993) 431-445.
Bodmer J., et al., Trends Biochem. Sci. 27(1), 19-26.
Broll, K., et al., Am. J. Clin. Pathol. 115, 543-549.
Buechele, C., et al., Eur. J. Immunol. 42, 737-748.
Choi, B.K., et al., J. Immunol. 182, 4107-4115.
Cuadros, C., et al., Int. J. Cancer 116, 934-943.
Curran, M.A., et al., PLoS One 6, e19499.
Diehl, L., et al., J. Immunol. 168, 3755-3762.
Dubrot, J., et al., Cancer Immunol. Immunother. 59, 1223-1233.
Futagawa, T., et al., Int. Immunol. 14, 275-286.
Guo, Z., et al., J Transl. Med. 11, 215.
Heinisch, I.V., et al., Eur. J. Immunol. 30, 3441-3446.
Hornig, N., et al., J. Immunother. 35, 418-429.
Ju, S.A., et al., Int. J. Cancer 122, 2784-2790.
Kienzle, G., and von Kempis, J. Int. Immunol. 12, 73-82.
Kim, D.H., et al., J. Immunol. 180, 2062-2068.
Kim, Y.H., et al., Mol. Cancer Ther. 8, 469-478.
Kwon, B.S., and Weissman, S.M. Proc. Natl. Acad. Sci USA 86, 1963-1967.
Lee, H., et al., J. Surg. Res. 169, e43-50.
Levitsky, V., et al., J. Immunol. 161, 594-601.
Li, F., and Ravetch, J.V., Science 333, 1030-1034.
Lin, W., et al., Blood 112, 699-707.
Melero, I., et al., Cell Immunol. 190, 167-172.
Melero, I., et al., Nat. Med. 3, 682-685.
Merchant, A.M., et al., Nat. Biotechnol. 16, 677-681.
Morales-Kastresana, A., et al., Clin. Cancer Res. 19, 6151-6162.
Mueller, D., et al., J. Immunother. 31, 714-722.
Murillo, O., et al., Eur. J. Immunol. 39, 2424-2436.
Narazaki, H., et al., Blood 115, 1941-1948.
Nishimoto, H., et al., Blood 106, 4241-4248.
Olofsson, P.S., et al., Circulation 117, 1292-1301.
Palazon, A., et al., Cancer Res. 71, 801-811.
Schwarz, H., et al., Blood 85, 1043-1052.
Shao, Z., and Schwarz, H. J. Leukoc. Biol. 89, 21-29.
Shi, W., and Siemann, D.W. Anticancer Res. 26, 3445-3453.
Simeone, E., and Ascierto, P.A. J Immunotoxicol. 9, 241-247.
Snell, L.M., et al., Immunol. Rev. 244, 197-217.
Stagg, J., et al., Proc. Natl. Acad. Sci USA 108, 7142-7147.
Teng, M.W., et al., J. Immunol. 183, 1911-1920.
von Kempis, J., et al., Osteoarthritis Cartilage 5, 394-406.
Wei, H., et al., PLoS One 8, e84927.
Wilcox, R.A., et al., J. Immunol. 168, 4262-4267.
Wilcox, R.A., et al., Blood 103, 177-184.
Zhang, N., et al., Clin. Cancer Res. 13, 2758-2767.
Zhang, X., et al., J. Immunol. 184, 787-795.

### Examples:

### Example 1

### General techniques

### Recombinant DNA techniques

Standard methods were used to manipulate DNA as described in Sambrook et al., Molecular Cloning: A Laboratory Manual, Second Edition, Cold Spring Harbor Laboratory Press, Cold Spring Harbor, N.Y, (1989). The molecular biological reagents were used according to the manufacturer's instructions.

### Gene synthesis

Desired gene segments were prepared by chemical synthesis at Geneart GmbH (Regensburg, Germany). The synthesized gene fragments were cloned into an E. coli plasmid for propagation/amplification. The DNA sequences of subcloned gene fragments were verified by DNA sequencing. Alternatively, short synthetic DNA fragments were assembled by annealing chemically synthesized oligonucleotides or via PCR. The respective oligonucleotides were prepared by metabion GmbH (Planegg-Martinsried, Germany).

### DNA sequence determination

DNA sequences were determined by double strand sequencing performed at MediGenomix GmbH (Martinsried, Germany) or SequiServe GmbH (Vaterstetten, Germany).

### DNA and protein sequence analysis and sequence data management

The EMBOSS (European Molecular Biology Open Software Suite) software package and Invitrogen's Vector NTI version 11.5 or Geneious prime were used for sequence creation, mapping, analysis, annotation and illustration.

### Reagents

All commercial chemicals, antibodies and kits were used as provided according to the manufacturer's protocol if not stated otherwise.

### Protein determination

The protein concentration of purified antibodies and derivatives was determined by determining the optical density (OD) at 280 nm, using the molar extinction coefficient calculated on the basis of the amino acid sequence according to Pace, et al., Protein Science 4 (1995) 2411-1423.

### Antibody concentration determination in supernatants

### 1) protein A beads

The concentration of antibodies in cell culture supernatants was estimated by immunoprecipitation with protein A agarose-beads (Roche Diagnostics GmbH, Mannheim, Germany). Therefore, 60 µL protein A Agarose beads were washed three times in TBS-NP40 (50 mM Tris buffer, pH 7.5, supplemented with 150 mM NaCl and 1% Nonidet-P40). Subsequently, 1-15 mL cell culture supernatant was applied to the protein A Agarose beads pre-equilibrated in TBS-NP40. After incubation for at 1 hour at room temperature the beads were washed on an Ultrafree-MC-filter column (Amicon) once with 0.5 mL TBS-NP40, twice with 0.5 mL 2x phosphate buffered saline (2xPBS, Roche Diagnostics GmbH, Mannheim, Germany) and briefly four times with 0.5 mL 100 mM Na-citrate buffer (pH 5.0). Bound antibody was eluted by addition of 35 µL NuPAGE^{®} LDS sample buffer (Invitrogen). Half of the sample was combined with NuPAGE^{®} sample reducing agent or left unreduced, respectively, and heated for 10 min. at 70 °C. Consequently, 5-30 µL were applied to a 4-12% NuPAGE^{®} Bis-Tris SDS-PAGE gel (Invitrogen) (with MOPS buffer for non-reduced SDS-PAGE and MES buffer with NuPAGE^{®} antioxidant running buffer additive (Invitrogen) for reduced SDS-PAGE) and stained with Coomassie Blue.

### 2) affinity HPLC

The concentration of the antibodies in cell culture supernatants was quantitatively measured by affinity HPLC chromatography. Briefly, cell culture supernatants containing antibodies that bind to protein A were applied to an Applied Biosystems Poros A/20 column in 200 mM KH₂PO₄, 100 mM sodium citrate, pH 7.4 and eluted with 200 mM NaCl, 100 mM citric acid, pH 2.5 on an Agilent HPLC 1100 system. The eluted antibody was quantified by UV absorbance and integration of peak areas. A purified standard IgG1 antibody served as a standard.

### 3) sandwich ELISA

The concentration of antibodies and derivatives in cell culture supernatants was measured by Sandwich-IgG-ELISA. Briefly, StreptaWell High Bind Streptavidin A-96 well microtiter plates (Roche Diagnostics GmbH, Mannheim, Germany) were coated with 100 µL/well biotinylated anti-human IgG capture molecule F(ab')2<h-Fcγ> BI (Dianova) at 0.1 µg/mL for 1 hour at room temperature or alternatively overnight at 4 °C and subsequently washed three times with 200 µL/well PBS, 0.05 % Tween (PBST, Sigma). Thereafter, 100 µL/well of a dilution series in PBS (Sigma) of the respective antibody containing cell culture supernatants was added to the wells and incubated for 1-2 hour on a shaker at room temperature. The wells were washed three times with 200 µL/well PBST and bound antibody was detected with 100 µL F(ab')2<hFcγ>POD (Dianova) at 0.1 µg/mL as the detection antibody by incubation for 1-2 hours on a shaker at room temperature. Unbound detection antibody was removed by washing three times with 200 µL/well PBST. The bound detection antibody was detected by addition of 100 µL ABTS/well followed by incubation. Determination of absorbance was performed on a Tecan Fluor Spectrometer at a measurement wavelength of 405 nm (reference wavelength 492 nm).

### Cultivation of CHO host cell line

CHO host cells were cultivated at 37 °C in a humidified incubator with 85 % humidity and 5 % CO₂. They were cultivated in a proprietary DMEM/F12-based medium containing 300 µg/mL Hygromycin B and 4 µg/mL of a second selection marker. The cells were splitted every 3 or 4 days at a concentration of 0.3×10E6 cells/ml in a total volume of 30 mL. For the cultivation 125 mL non-baffle Erlenmeyer shake flasks were used. Cells were shaken at 150 rpm with a shaking amplitude of 5 cm. The cell count was determined with Cedex HiRes Cell Counter (Roche Diagnostics GmbH, Mannheim, Germany). Cells were kept in culture until they reached an age of 60 days.

### Transformation 10-beta competent E. coli cells

For transformation, the 10-beta competent E. coli cells were thawed on ice. After that, 2 µL of plasmid DNA were pipetted directly into the cell suspension. The tube was flicked and put on ice for 30 minutes. Thereafter, the cells were placed into the 42 °C-warm thermal block and heat-shocked for exactly 30 seconds. Directly afterwards, the cells were chilled on ice for 2 minutes. 950 µL of NEB 10-beta outgrowth medium were added to the cell suspension. The cells were incubated under shaking at 37 °C for one hour. Then, 50-100 µL were pipetted onto a pre-warmed (37 °C) LB-Amp agar plate and spread with a disposable spatula. The plate was incubated overnight at 37 °C. Only bacteria, which have successfully incorporated the plasmid, carrying the resistance gene against ampicillin, can grow on these plates. Single colonies were picked the next day and cultured in LB-Amp medium for subsequent plasmid preparation.

### Bacterial culture

Cultivation of E. coli was done in LB-medium, short for Luria Bertani, which was spiked with 1 mL/L 100 mg/mL ampicillin resulting in an ampicillin concentration of 0.1 mg/mL. For the different plasmid preparation quantities, the following amounts were inoculated with a single bacterial colony.

**Table: E. coli cultivation volumes**

| **Quantity plasmid preparation** | **Volume LB-Amp medium [mL]** | **Incubation time [h]** |
|---|---|---|
| Mini-Prep 96-well (EpMotion) | 1.5 | 23 |
| Mini-Prep 15 mL-tube | 3.6 | 23 |
| Maxi-Prep | 200 | 16 |

For Mini-Prep, a 96-well 2 mL deep-well plate was filled with 1.5 mL LB-Amp medium per well. The colonies were picked and the toothpick was tuck in the medium. When all colonies were picked, the plate closed with a sticky air porous membrane. The plate was incubated in a 37 °C incubator at a shaking rate of 200 rpm for 23 hours.

For Mini-Preps a 15 mL-tube (with a ventilated lid) was filled with 3.6 mL LB-Amp medium and equally inoculated with a bacterial colony. The toothpick was not removed but left in the tube during incubation. Like the 96-well plate, the tubes were incubated at 37 °C, 200 rpm for 23 hours.

For Maxi-Prep 200 mL of LB-Amp medium were filled into an autoclaved glass 1 L Erlenmeyer flask and inoculated with 1 mL of bacterial day-culture, which was roundabout 5 hours old. The Erlenmeyer flask was closed with a paper plug and incubated at 37 °C, 200 rpm for 16 hours.

### Plasmid preparation

For Mini-Prep, 50 µL of bacterial suspension were transferred into a 1 mL deep-well plate. After that, the bacterial cells were centrifuged down in the plate at 3000 rpm, 4 °C for 5 min. The supernatant was removed and the plate with the bacteria pellets placed into an EpMotion. After approx. 90 minutes, the run was done and the eluted plasmid-DNA could be removed from the EpMotion for further use.

For Mini-Prep, the 15 mL tubes were taken out of the incubator and the 3.6 mL bacterial culture splitted into two 2 mL Eppendorf tubes. The tubes were centrifuged at 6,800xg in a table-top microcentrifuge for 3 minutes at room temperature. After that, Mini-Prep was performed with the Qiagen QIAprep Spin Miniprep Kit according to the manufacturer's instructions. The plasmid DNA concentration was measured with Nanodrop.

Maxi-Prep was performed using the Macherey-Nagel NucleoBond^{®} Xtra Maxi EF Kit according to the manufacturer's instructions. The DNA concentration was measured with Nanodrop.

### Ethanol precipitation

The volume of the DNA solution was mixed with the 2.5-fold volume ethanol 100 %. The mixture was incubated at -20 °C for 10 min. Then the DNA was centrifuged for 30 min. at 14,000 rpm, 4 °C. The supernatant was carefully removed and the pellet washed with 70 % (v/v) ethanol. Again, the tube was centrifuged for 5 min. at 14,000 rpm, 4 °C. The supernatant was carefully removed by pipetting and the pellet dried. When the ethanol was evaporated, an appropriate amount of endotoxin-free water was added. The DNA was given time to re-dissolve in the water overnight at 4 °C. A small aliquot was taken and the DNA concentration was measured with a Nanodrop device.

### Preparative antibody purification

Antibodies were purified from filtered cell culture supernatants referring to standard protocols. In brief, antibodies were applied to a protein A Sepharose column (GE healthcare) and washed with PBS. Elution of antibodies was achieved at pH 2.8 followed by immediate neutralization. Aggregated protein was separated from monomeric antibodies by size exclusion chromatography (Superdex 200, GE Healthcare) in PBS or in 20 mM Histidine buffer comprising 150 mMNaCl (pH 6.0). Monomeric antibody fractions were pooled, concentrated (if required) using e.g., a MILLIPORE Amicon Ultra (30 MWCO) centrifugal concentrator, frozen and stored at -20 °C or -80 °C. Part of the samples were provided for subsequent protein analytics and analytical characterization e.g. by SDS-PAGE, size exclusion chromatography (SEC) or mass spectrometry.

### SDS-PAGE

The NuPAGE^{®} Pre-Cast gel system (Invitrogen) was used according to the manufacturer's instruction. In particular, 10 % or 4-12 % NuPAGE^{®} Novex^{®} Bis-TRIS Pre-Cast gels (pH 6.4) and a NuPAGE^{®} MES (reduced gels, with NuPAGE^{®} antioxidant running buffer additive) or MOPS (non-reduced gels) running buffer was used.

### CE-SDS

Purity and antibody integrity were analyzed by CE-SDS using microfluidic Labchip technology (PerkinElmer, USA). Therefore, 5 µL of antibody solution was prepared for CE-SDS analysis using the HT Protein Express Reagent Kit according manufacturer's instructions and analyzed on Labchip GXII system using a HT Protein Express Chip. Data were analyzed using Labchip GX Software.

### Analytical size exclusion chromatography

Size exclusion chromatography (SEC) for the determination of the aggregation and oligomeric state of antibodies was performed by HPLC chromatography. Briefly, protein A purified antibodies were applied to a Tosoh TSKgel G3000SW column in 300 mM NaCl, 50 mM KH₂PO₄/K₂HPO₄ buffer (pH 7.5) on an Dionex Ultimate^{®} system (Thermo Fischer Scientific), or to a Superdex 200 column (GE Healthcare) in 2 × PBS on a Dionex HPLC-System. The eluted antibody was quantified by UV absorbance and integration of peak areas. BioRad Gel Filtration Standard 151-1901 served as a standard.

### Mass spectrometry

This section describes the characterization of the antibodies/fusion proteins with emphasis on their correct assembly. The expected primary structures were analyzed by electrospray ionization mass spectrometry (ESI-MS) of the deglycosylated intact antibody and in special cases of the deglycosylated/limited LysC digested antibody.

The antibodies/fusion proteins were deglycosylated with N-Glycosidase F in a phosphate or Tris buffer at 37 °C for up to 17 h at a protein concentration of 1 mg/mL. The limited LysC (Roche Diagnostics GmbH, Mannheim, Germany) digestions were performed with 100 µg deglycosylated antibody in a Tris buffer (pH 8) at room temperature for 120 hours, or at 37 °C for 40 min., respectively. Prior to mass spectrometry the samples were desalted via HPLC on a Sephadex G25 column (GE Healthcare). The total mass was determined via ESI-MS on a maXis 4G UHR-QTOF MS system (Bruker Daltonik) equipped with a TriVersa NanoMate source (Advion).

### Example 2

### Plasmid generation for random integration

For the expression of the antibodies/fusion proteins, expression vectors for transient expression (e.g. in HEK293 cells) based either on a cDNA organization with or without a CMV-intron A promoter or on a genomic organization with a CMV promoter can be applied.

### Expression cassette composition

For the expression of an antibody chain, a transcription unit comprising the following functional elements was used:
- the immediate early enhancer and promoter from the human cytomegalovirus including intron A,
- a human heavy chain immunoglobulin 5'-untranslated region (5'UTR),
- a murine immunoglobulin heavy chain signal sequence,
- a nucleic acid encoding the respective antibody chain,
- the bovine growth hormone polyadenylation sequence (BGH pA), and
- optionally the human gastrin terminator (hGT).

Beside the expression unit/cassette including the desired gene to be expressed, the basic/standard mammalian expression plasmid contains
- an origin of replication from the vector pUC18 which allows replication of this plasmid in E. coli, and
- a beta-lactamase gene which confers ampicillin resistance in E. coli.

The fusion genes encoding the antibody chains are generated by PCR and/or gene synthesis and assembled by known recombinant methods and techniques by connection of the according nucleic acid segments e.g. using unique restriction sites in the respective vectors. The subcloned nucleic acid sequences are verified by DNA sequencing. For transient transfections, larger quantities of the vectors are prepared by vector preparation from transformed E. coli cultures (NucleoBond AX, Macherey-Nagel).

For all constructs knob-into-hole heterodimerization technology was used with a typical knob (T366W) substitution in the first CH3 domain and the corresponding hole substitutions (T366S, L368A and Y407V) in the second CH3 domain (as well as two additional introduced cysteine residues S354C/Y349C) (contained in the respective corresponding heavy chain (HC) sequences depicted above).

### Example 3

### Transient expression

### HEK 293 cells

Transient expression was performed in suspension-adapted HEK293F (FreeStyle 293-F cells; Invitrogen) cells with Transfection Reagent 293-free (Novagen).

Cells have been passaged, by dilution, at least four times (volume 30 mL) after thawing in a 125 mL shake flask (Incubate/Shake at 37 °C, 7 % CO₂, 85 % humidity, 135 rpm).

The cells were expanded to 3×10E5 cells/mL in 250 mL volume. Three days later, cells have been split and new seeded with a density of 7^{∗}10⁵ cells/mL in a 250 mL volume in a 1-liter shake flask. Transfection will be 24 hours later at a cell density around 1.4 - 2.0 × 10⁶ cells/mL.

Before transfection 250 µg plasmid-DNA were diluted in a final volume of 10 mL with pre-heated (water bath; 37 °C) Opti-MEM (Gibco). The solution was gently mixed and incubated at room temperature for not longer than 5 min. Then 333.3 µL 293-free transfection reagent were added to the DNA-OptiMEM-solution. Thereafter the solution was gently mixed and incubated at room temperature for 15-20 minutes.

The whole volume of mixture was added to 1 L shake flask. The cells were incubated at 37 °C, 7 % CO₂, 85 % humidity, 135 rpm for 6 or 7 days.

The supernatant was harvested by a first centrifugation-step at 2,000 rpm, 4 °C, for 10 minutes. Then the supernatant was transferred into a new centrifugation-flask for a second centrifuge at 4,000 rpm, 4 °C, for 20 minutes. Thereafter the cell-free-supernatant was filtered through a 0.22 µm bottle-top-filter and stored in a freezer (-20 °C).

### CHO-K1 cells

Transient expression was performed in suspension-adapted CHO-K1 cells with transfection reagent Nucleofector solution V (Lonza) and Amaxa nucleoporator for electroporation.

Cells have been passaged, by dilution, at least four times (volume 30 mL) after thawing in a 125 mL shake flask (incubation at 37 °C, 7 % CO₂, 85 % humidity, 140 rpm).

The cells were expanded to 3×10⁵ cells/mL in 60 mL volume in a 250 mL shake flask. Cells will be ready for transfection at a cell density around 1.2-2.0 × 10⁶ cells/mL. Total amount of 1×10⁷ cells were collected by centrifugation at 1000 rpm, room temperature for 10 minutes. The cell pellet was dissolved in 100 µL pre-warmed (RT) Nucleofector solution (Lonza). 1.2 pmol in total or 10 µg maximum plasmid DNA (diluted in water) was mixed in a final volume of 10 µL, followed by mixing with 100 µL transfection reagent and 1×10⁷ cells. The mixture of plasmid DNA, transfection reagent and cells was then transferred into the electroporation cuvette. Directly without any incubation time, the cuvette was placed into the Nucleofector system. After electroporation with program "U-24", 500 µL of pre-warmed (37 °C) medium was added to the cuvette. The whole mixture was transferred to 125 mL shake flask with 30 mL pre-warmed (37 °C) chemically defined medium (Invitrogen).

### CHO-K1 TI cells

Transient expression was performed in suspension-adapted CHO-K1 TI-host cells with transfection reagent PE buffer and MaxCyte (OC-400 processing assembly) for electroporation.

Cells have been passaged, at least four times after thawing in a 125 mL shake flask (incubation at 37 °C, 5 % CO₂, 85 % humidity, 150 rpm).

The cells were expanded to 4×10⁵ cells/mL in a shake flask on the first day. On the third day, the cells will be ready for transfection at a cell density around 1-2 × 10⁶ cells/mL.

Total amount of 3×10⁶ cells were collected by centrifugation at 1000 rpm, room temperature for 10 minutes. The cell pellet was dissolved in 300 µL PE buffer (MaxCyte), followed by adding of a maximum of 25 µg plasmid DNA. The mixture of plasmid DNA, transfection reagent and cells was then transferred into the electroporation cuvette followed by electroporation using "CHO-2" and process assemblies protocols. After electroporation, the whole mixture was transferred to shake flask at 37 °C degree for 30 minutes without agitation. After 30 minutes, 30 mL recovery medium is added.

The transfected cells were incubated with shaking at 37 °C, 5 % CO₂, 85 % humidity, 100 rpm for 7 days.

### ExpiCHO cells

Transient expression was performed with the ExpiCHO-S Expression System (A29133; Gibco).

Thawing, passaging and the transfection were done according to the manufacturer's instructions (see ExpiCHO Expression System (A29133) User guide). For the transfection the "standard Protocol" (ref. page 12 A29133, manual) was used.

The transfected cells were incubated with shaking at 37 °C, 7 % CO₂, 85 % humidity, 140 rpm for 7 days.

The supernatant was harvested by a first centrifugation-step at 1,000 rpm, 4 °C, for 10 minutes. The supernatant was transferred into a new centrifugation-flask for a second centrifuge at 4,000 rpm, 4 °C, for 20 minutes. Thereafter the cell-free-supernatant was filtered through a 0.22 µm bottle-top-filter and stored in a freezer (-20 °C) until further use.

### Example 4

### Stable expression and purification

### Stable cell line generation

Two double plasmids and a single plasmid were used to co-transfect the host cell line CHO K1-M. The double plasmids contained DNA fragments coding for VL, VH of FAP and monomer and dimer of 4-1-BBL fusion protein as well as the CH and CL of FAP and 4-1-BBL while the single plasmid contained only the dimer of 4-1-BBL. All sequences were chemically synthesized and thereby combined with heterologous DNA elements, which include: a) 5'-UTR including a 5'-Kozak sequence, b) a DNA segment coding for a leader sequence (LL), c) suitable restriction endonuclease sites for cloning which are added at the 5'- and 3'-end of DNA segments to be synthesize.

The host cell line is derived from the proline auxotrophic strain K1 (Kao and Puck, 1967), which was established from the CHO cell line introduced by Puck et al. CHO K1 cells were obtained from the American Type Culture Collection (ATCC) as frozen stocks (registration number CCL-61), subsequently adapted to growth in a chemically defined medium and suspension at Roche Pharma, Penzberg, and then denoted as "CHO K1-M".

One ampoule of the CHO K1-M WCB was used for transfection. The transfection was performed using linearized DNA in a chemically defined medium. Clones that stably integrated the recombinant DNA were selected based on the DHFR/MTX expression system. For selection of stable transfectants, the cells were transferred to 384-well plates at a density of 500 cells/well and cultivated in the chemically defined medium containing 400 nM MTX. These conditions assured that only cells that overexpressed the DHFR gene from double and single plasmids survived.

Three weeks after transfection the supernatants were screened for the presence of human IgG by an anti-human Fc ELISA. Clones positive for antibody titers were expanded to the 96 well format, and MTX concentration was then decreased to 250 nmol/L MTX. One week later, clones were tested by ELISA for binding to the FAP antigen and to the 4-1-BB receptor in order to ensure the functionality of the produced FAP antibody-4-1-BBL fusion protein molecules. Positive clones were expanded and banked.

### Purification of the antibodies

The antibody-containing culture supernatants were filtered and purified by two chromatographic steps. The antibodies were captured by affinity chromatography using HiTrap MabSelectSuRe (GE Healthcare) equilibrated with PBS (1 mM KH₂PO₄, 10 mM Na₂HPO₄, 137 mM NaCl, 2.7 mM KCl), pH 7.4. Unbound proteins were removed by washing with equilibration buffer, and the antibody was recovered with 50 mM citrate buffer, pH 2.8, and immediately after elution neutralized to pH 6.0 with 1 M Tris-base, pH 9.0. Size exclusion chromatography on Superdex 200TM (GE Healthcare) was used as second purification step. The size exclusion chromatography was performed in 20 mM histidine buffer, 0.14 M NaCl, pH 6.0. The antibody containing solutions were concentrated with an Ultrafree-CL centrifugal filter unit equipped with a Biomax-SK membrane (Millipore, Billerica, MA) and stored at -80 °C.

### Example 5

### Plasmid generation for targeted integration

To construct two-plasmid antibody constructs, the respective structural genes were cloned into a front vector backbone containing L3 and LoxFas sequences, and a back vector containing LoxFas and 2L sequences and a pac selectable marker. A Cre recombinase plasmid (see, e.g., Wong, E.T., et al., Nucl. Acids Res. 33 (2005) e147; O'Gorman, S., et al., Proc. Natl. Acad. Sci. USA 94 (1997) 14602-14607) was used for all RMCE processes. See also WO 2019/126634, which is incorporated herein by reference in its entirety.

The cDNAs encoding the respective polypeptides were generated by gene synthesis (Geneart, Life Technologies Inc.). The gene synthesis and the backbone-vectors were digested with HindIII-HF and EcoRI-HF (NEB) at 37 °C for 1 h and separated by agarose gel electrophoresis. The DNA-fragment of the insert and backbone were cut out from the agarose gel and extracted by QIAquick Gel Extraction Kit (Qiagen). The purified insert and backbone fragment was ligated via the Rapid Ligation Kit (Roche Diagnostics GmbH, Mannheim, Germany) following the manufacturer's protocol with an Insert/Backbone ratio of 3:1. The ligation approach was then transformed in competent E.coli DH5α via heat shock for 30 sec. at 42 °C and incubated for 1 h at 37 °C before they were plated out on agar plates with ampicillin for selection. Plates were incubated at 37 °C overnight.

On the following day clones were picked and incubated overnight at 37 °C under shaking for the Mini or Maxi-Preparation, which was performed with the EpMotion^{®} 5075 (Eppendorf) or with the QIAprep Spin Mini-Prep Kit (Qiagen)/ NucleoBond Xtra Maxi EF Kit (Macherey & Nagel), respectively. All constructs were sequenced to ensure the absence of any undesirable mutations.

In the second cloning step, the previously cloned vectors were digested with KpnI-HF/SalI-HF and SalI-HF/MfeI-HF with the same conditions as for the first cloning. The TI backbone vector was digested with KpnI-HF and MfeI - HF. Separation and extraction was performed as described above. Ligation of the purified insert and backbone was performed using T4 DNA Ligase (NEB) following the manufacturing protocol with an Insert/Insert/Backbone ratio of 1:1:1 overnight at 4 °C and inactivated at 65 °C for 10 min. The following cloning steps were performed as described above.

### Example 6

### Generation of stable cell lines by targeted integration

CHO-K1 TI host cells comprising a GFP expression cassette in the TI landing site were propagated in disposable 125 mL vented shake flasks under standard humidified conditions (95 % rH, 37 °C, and 5 % CO₂) at a constant agitation rate of 150 rpm in a proprietary DMEM/F12-based medium. Every 3-4 days the cells were seeded in chemically defined medium containing selection marker 1 and selection marker 2 in effective concentrations with a concentration of 3×10E5 cells/mL. Density and viability of the cultures were measured with a Cedex HiRes cell counter (F. Hoffmann-La Roche Ltd, Basel, Switzerland).

For stable transfection, equimolar amounts of front and back vector were mixed. 1 µg Cre expression plasmid was added per 5 µg of the mixture, i.e. 5 µg Cre expression plasmid or Cre mRNA was added to 25 µg of the front- and back-vector mixture.

Two days prior to transfection TI host cells were seeded in fresh medium with a density of 4×10E5 cells/mL. Transfection was performed with the Nucleofector device using the Nucleofector Kit V (Lonza, Switzerland), according to the manufacturer's protocol. 3×10E7 cells were transfected with a total of 30 µg nucleic acids, i.e. with 30 µg plasmid (5 µg Cre plasmid and 25 µg front- and back-vector mixture). After transfection, the cells were seeded in 30 mL medium without selection agents.

On day 5 after seeding the cells were centrifuged and transferred to 80 mL chemically defined medium containing puromycin (selection agent 1) and 1-(2'-deoxy-2'-fluoro-1-beta-D-arabinofuranosyl-5-iodo)uracil (FIAU; selection agent 2) at effective concentrations of 6×10E5 cells/ml for selection of recombinant cells. The cells were incubated at 37 °C, 150 rpm. 5 % CO₂, and 85 % humidity from this day on without splitting. Cell density and viability of the culture was monitored regularly. When the viability of the culture started to increase again, the concentrations of selection agents 1 and 2 were reduced to about half the amount used before. In more detail, to promote the recovering of the cells, the selection pressure was reduced if the viability is > 40 % and the viable cell density (VCD) is > 0.5×10E6 cells/mL. Therefore, 4×10E5 cells/mL were centrifuged and re-suspended in 40 ml selection media II (chemically-defined medium, ½ selection marker 1 & 2). The cells were incubated with the same conditions as before and also not splitted.

Ten days after starting selection, the success of Cre mediated cassette exchange was checked by flow cytometry measuring the expression of intracellular GFP and extracellular heterologous fusion polypeptide bound to the cell surface. An APC antibody (allophycocyanin-labeled F(ab')2 fragment goat anti-human IgG) against human antibody light and heavy chain was used for FACS staining. Flow cytometry was performed with a BD FACS Canto II flow cytometer (BD, Heidelberg, Germany). Ten thousand events per sample were measured. Living cells were gated in a plot of forward scatter (FSC) against side scatter (SSC). The live cell gate was defined with non-transfected TI host cells and applied to all samples by employing the FlowJo 7.6.5 EN software (TreeStar, Olten, Switzerland). Fluorescence of GFP was quantified in the FITC channel (excitation at 488 nm, detection at 530 nm). Heterologous fusion polypeptide was measured in the APC channel (excitation at 645 nm, detection at 660 nm). Parental CHO cells, i.e. those cells used for the generation of the TI host cell, were used as a negative control with regard to GFP and fusion polypeptide expression. Fourteen days after the selection had been started, the viability exceeded 90 % and selection was considered as complete.

### Example 7

### FACS screening

FACS analysis was performed to check the transfection efficiency and the RMCE efficiency of the transfection. 4×10E5 cells of the transfected approaches were centrifuged (1200 rpm, 4 min.) and washed twice with 1 mL PBS. After the washing steps with PBS the pellet was re-suspended in 400 µL PBS and transferred in FACS tubes (Falcon ^{®} Round-Bottom Tubes with cell strainer cap; Corning). The measurement was performed with a FACS Canto II and the data were analyzed by the software FlowJo.

### Example 8

### Fed-batch cultivation

Fed-batch production cultures were performed in shake flasks or Ambr15 vessels (Sartorius Stedim) with proprietary chemically defined medium. Cells were seeded at 1×10E6 cells/mL on day 0. Cultures received proprietary feed medium on days 3, 7, and 10. Viable cell count (VCC) and percent viability of cells in culture was measured on days 0, 3, 7, 10, and 14 using a Cedex HiRes instrument (Roche Diagnostics GmbH, Mannheim, Germany). Glucose, lactate and product titer concentrations were measured on days 3, 5, 7, 10, 12 and 14 using a Cobas Analyzer (Roche Diagnostics GmbH, Mannheim, Germany). The supernatant was harvested 14 days after start of fed-batch by centrifugation (10 min., 1,000 rpm and 10 min., 4,000 rpm) and cleared by filtration (0.22 µm). Day 14 titers were determined using protein A affinity chromatography with UV detection. Product quality was determined by Caliper's Labchip (Caliper Life Sciences).

### Example 9

### Fusion polypeptide quantification

The titers in the culture medium were measured with an anti-human IgG sandwich ELISA. In brief, the fusion polypeptide was captured from the cell culture fluid with an anti-human Fc antibody bound to a MaxiSorp microtiter plate (Nunc^{™}, Sigma-Aldrich) and detected with an anti-human Fc antibody-POD conjugate, which binds

## Claims

1. A method for producing an antibody-multimer-fusion polypeptide
comprising
(a) an antibody heavy chain and an antibody light chain, and
(b) a first fusion polypeptide comprising in N- to C-terminal direction a first part of a non-antibody multimeric polypeptide, an antibody heavy chain CH1 domain or an antibody light chain constant domain, an antibody hinge region, an antibody heavy chain CH2 domain and an antibody heavy chain CH3 domain, and a second fusion polypeptide comprising in N- to C-terminal direction the second part of the non-antibody multimeric polypeptide and an antibody light chain constant domain if the first polypeptide comprises an antibody heavy chain CH1 domain or an antibody heavy chain CH1 domain if the first polypeptide comprises an antibody light chain constant domain,
wherein
(i) the antibody heavy chain of (a) and the first fusion polypeptide of (b), (ii) the antibody heavy chain of (a) and the antibody light chain of (a), and (iii) the first fusion polypeptide of (b) and the second fusion polypeptide of (b) are each independently of each other covalently linked to each other by at least one disulfide bond,
wherein
the variable domains of the antibody heavy chain and the antibody light chain form a binding site specifically binding to an antigen,
**characterized in that** the antibody-multimer-fusion polypeptide is expressed by a recombinant mammalian cell obtained by transfecting a (parent) mammalian cell with the expression cassettes for the antibody heavy chain, the antibody light chain, the first fusion polypeptide and the second fusion polypeptide at a stoichiometric ratio of 1:1:2:1.

2. The method according to claim 1, wherein the mammalian cell is a CHO cell or a HEK cell.

3. The method according to any one of claims 1 to 2, wherein the transfecting is of three vectors, whereby the first vector comprises the expression cassettes for the an antibody heavy chain and an antibody light chain, the second expression vector comprises the expression cassettes for the first fusion polypeptide and the second fusion polypeptide, and the third vector comprises one expression cassette for the first fusion polypeptide.

4. The method according to any one of claims 1 to 3, wherein the first fusion polypeptide comprises as first part of the non-antibody multimeric polypeptide two ectodomains of a TNF ligand family member or a fragment thereof that are connected to each other by a peptide linker, and the second fusion polypeptide comprises as second part of a non-antibody multimeric polypeptide only one ectodomain of said TNF ligand family member or a fragment thereof, or vice versa.

5. The method according to claim 4, wherein
(a) the first fusion polypeptide comprises as first part of the non-antibody multimeric polypeptide a first ectodomain of a TNF ligand family member or a fragment thereof, a spacer domain and a second ectodomain of said TNF ligand family member or a fragment thereof, wherein
- the spacer domain is a polypeptide and comprises at least 25 amino acid residues,
- the first ectodomain of a TNF ligand family member or a fragment thereof is fused either directly or via a first peptide linker to the N-terminus of the spacer domain and
- the second ectodomain of said TNF ligand family member or a fragment thereof is fused either directly or via a second peptide linker to the C-terminus of the spacer domain,
(b) the second fusion polypeptide comprises as second part of the non-antibody multimeric polypeptide a third ectodomain of said TNF ligand family member or a fragment thereof that is fused either directly or via a third peptide linker to
- either the C-terminus of the second ectodomain of said TNF ligand family member in the first fusion polypeptide or to the C-terminus of the spacer domain in the second fusion polypeptide, or
- in case the second part of the antigen binding domain is fused to the C-terminus of the spacer domain of the second fusion protein, to the C-terminus of the second ectodomain of said TNF ligand family member in the first fusion polypeptide.

6. The method according to any one of claims 1 to 5, wherein
the first fusion polypeptide comprises in N- to C-terminal direction a first part of a non-antibody multimeric polypeptide, an antibody light chain constant domain, an antibody hinge region, an antibody heavy chain CH2 domain and an antibody heavy chain CH3 domain, and
the second fusion polypeptide comprising in N- to C-terminal direction the second part of the non-antibody multimeric polypeptide and an antibody heavy chain CH1 domain.

7. The method according to any one of claims 1 to 6, wherein
the first fusion polypeptide comprises the knob mutation, and
the antibody heavy chain comprises the hole mutations.

8. The method according to any one of claims 1 to 7, wherein the antibody heavy chain of (a) and the first fusion polypeptide of (b) form an Fc-region.

9. The method according to any one of claims 4 to 8, wherein in the first fusion polypeptide the two ectodomains of a TNF ligand family member or fragments thereof connected to each other by a first peptide linker and are fused at its C-terminus by a second peptide linker to a CH1 domain, and in the second fusion polypeptide the one ectodomain of said TNF ligand family member or a fragment thereof is fused at its C-terminus by a third peptide linker to the antibody light chain constant domain.

10. The method according to any one of claims 4 to 8, wherein in the first fusion polypeptide the two ectodomains of a TNF ligand family member or fragments thereof connected to each other by a first peptide linker and are fused at its C-terminus by a second peptide linker to light chain constant domain, and in the second fusion polypeptide the one ectodomain of said TNF ligand family member or a fragment thereof is fused at its C-terminus by a third peptide linker to the a heavy chain CH1 domain.

11. The method according to any one of claims 1 to 10, wherein the variable domains of the antibody heavy chain and the antibody light chain form a binding site specifically binding to a cell surface antigen selected from the group consisting of Fibroblast Activation Protein (FAP), Melanoma-associated Chondroitin Sulfate Proteoglycan (MCSP), Epidermal Growth Factor Receptor (EGFR), Carcinoembryonic Antigen (CEA), CD19, CD20 and CD33.

12. The method according to any one of claims 4 to 11, wherein the TNF ligand family member is selected from 4-1-BBL and OX40L.

13. The method according to any one of claims 1 to 12, wherein
(a) the antibody heavy chain and the antibody light chain form a binding site capable of specific binding to a target cell antigen, and
(b) the first fusion polypeptide comprises an amino acid sequence selected from the group consisting of SEQ ID NO: 05, SEQ ID NO: 57, SEQ ID NO: 58 and SEQ ID NO: 59, and the second fusion polypeptide comprises an amino acid sequence selected from the group consisting of SEQ ID NO: 01, SEQ ID NO: 56, SEQ ID NO: 03 and SEQ ID NO: 04.

14. The method according to any one of claims 1 to 13, wherein the variable domains of the antibody heavy chain and the antibody light chain form a binding site specifically binding to FAP and comprise a VH domain comprising (i) CDR-H1 comprising the amino acid sequence of SEQ ID NO: 06 or SEQ ID NO: 60, (ii) CDR-H2 comprising the amino acid sequence of SEQ ID NO: 07 or SEQ ID NO: 61, and (iii) CDR-H3 comprising the amino acid sequence of SEQ ID NO: 08 or SEQ ID NO: 62, and a VL domain comprising (iv) CDR-L1 comprising the amino acid sequence of SEQ ID NO: 09 or SEQ ID NO: 63, (v) CDR-L2 comprising the amino acid sequence of SEQ ID NO: 10 or SEQ ID NO: 64, and (vi) CDR-L3 comprising the amino acid sequence of SEQ ID NO: 11 or SEQ ID NO: 65.

15. The method according to any one of claims 1 to 13, wherein the variable domains of the antibody heavy chain and the antibody light chain form a binding site specifically binding to CEA and comprise a VH domain comprising (i) CDR-H1 comprising the amino acid sequence of SEQ ID NO: 84, (ii) CDR-H2 comprising the amino acid sequence of SEQ ID NO: 85, and (iii) CDR-H3 comprising the amino acid sequence of SEQ ID NO: 86, and a VL domain comprising (iv) CDR-L1 comprising the amino acid sequence of SEQ ID NO: 87, (v) CDR-L2 comprising the amino acid sequence of SEQ ID NO: 88, and (vi) CDR-L3 comprising the amino acid sequence of SEQ ID NO: 89.

16. The method according to any one claims 1 to 13, wherein the variable domains of the antibody heavy chain and the antibody light chain form a binding site specifically binding to CD19 and comprise a VH domain comprising (i) CDR-H1 comprising the amino acid sequence of SEQ ID NO: 104 or SEQ ID NO: 105, (ii) CDR-H2 comprising the amino acid sequence of SEQ ID NO: 106 or SEQ ID NO: 107, and (iii) CDR-H3 comprising the amino acid sequence of SEQ ID NO: 108 or SEQ ID NO: 109, and a VL domain comprising (iv) CDR-L1 comprising the amino acid sequence of SEQ ID NO: 110 or SEQ ID NO: 111, (v) CDR-L2 comprising the amino acid sequence of SEQ ID NO: 112 or SEQ ID NO: 113, and (vi) CDR-L3 comprising the amino acid sequence of SEQ ID NO: 114 or SEQ ID NO: 115.
